# EUROPÄISCHE PATENTSCHRIFT

(11) **EP 3 087 230 B1**
(45) Veröffentlichungstag und Bekanntmachung des Hinweises auf die Patenterteilung: **07.03.2018**
(21) Anmeldenummer: 15771145.8
(22) Anmeldetag: 30.09.2015
(51) Int. Cl.: E04F 13/08, E04F 15/10, E04C 2/38, E04F 15/02

(54) **PANEEL**
PANEL
PANNEAU

(30) Priorität: 30.09.2014 DE 102014114250
(43) Veröffentlichungstag der Anmeldung: 02.11.2016
(60) Teilanmeldung: 17208365.1
(73) Patentinhaber: Akzenta Paneele + Profile GmbH, 56759 Kaisersesch (DE)
(72) Erfinder: HANNIG, Hans-Jürgen, 51427 Bergisch Gladbach (DE); SCHÄFERS, Erich, 54552 Demerath (DE)
(74) Vertreter: Lippert Stachow Patentanwälte Rechtsanwälte
(86) Internationale Anmeldenummer: PCT/EP2015/072564
(87) Internationale Veröffentlichungsnummer: WO 2016/050848

(56) Entgegenhaltungen:
- EP-A2- 0 220 389
- DE-U1-202014 010 455
- US-A1- 2010 018 149
- US-A1- 2011 167 744

## Beschreibung

Die Erfindung betrifft ein Paneel mit wenigstens einem Paar komplementärer Verriegelungsmittel an gegenüberliegenden Paneelkanten, wobei die Verriegelungsmittel als formschlüssige Halteprofile mit Verriegelungsnut beziehungsweise mit komplementärer Verriegelungsfeder ausgestaltet sind, wobei die Paneeloberfläche wenigstens am Rand eines der Halteprofile eine Kantenbrechung aufweist, mit der Maßgabe, dass jenes mit der Kantenbrechung versehene Halteprofil unterhalb der Kantenbrechung mit einer oberen Stoßfläche versehen ist, und das komplementäre Halteprofil mit einer dazu im Wesentlichen parallel angeordneten komplementären oberen Stoßfläche versehen ist, und wobei durch die beiden Stoßflächen im Kontakt miteinander eine Stoßfuge erzeugbar ist, wobei die Stoßfuge relativ zur Paneeloberfläche geneigt ist, und zu diesem Zweck eine der Stoßflächen einer Feder zugeordnet ist und in Richtung des freien Endes der betreffenden Feder abwärts geneigt ist und die komplementäre obere Stoßfläche einer Nut zugeordnet ist und in Richtung des Grundes der betreffenden Nut abwärts geneigt ist.

Mit derlei Paneelen werden beispielsweise Fußbodenbeläge hergestellt, insbesondere eignet es sich für schwimmend verlegte Fußbodenbeläge. Die Paneele weisen üblicherweise dekorative Oberflächen auf.

Aus der DE 20 2008 011 589 ist ein Paneel bekannt, das obere Stoßflächen aufweist, zwischen welchen sich ein Spalt bilden kann. Es wird eine Kantenbrechung vorgeschlagen, die bewirkt, dass ein entstandener Spalt weniger augenfällig ist als bei einem Fußbodenbelag aus Paneelen ohne Kantenbrechung, welche eine ebene Fußbodenoberfläche ergeben, so dass sich Spalte deutlich sichtbar zeigen. Durch die Kantenbrechung der Paneele entsteht beispielsweise eine V-Fuge. Ein Spalt zeigt sich dann nicht an der Paneeloberfläche, sondern an der tiefsten Stelleeiner solchen V-Fuge und ist dann je nach Blickrichtung von oben gar nicht sichtbar für einen Betrachter. Dadurch wird das Erscheinungsbild eines Fußbodenbelags aufgrund der V-Fuge beziehungsweise der Kantenbrechung weniger gestört als bei einem Fußbodenbelag aus Paneelen, die keine Kantenbrechung aufweisen und Spalte an der Oberfläche eines Fußbodenbelags deutlich sichtbar sind.

Ein gattungsgemäßes Paneel ist aus der US 2010/0018149 A1 bekannt, und zwar das Ausführungsbeispiel gemäß Fig. 12. Darin wird ein Paneel vorgeschlagen, das komplementäre Verriegelungsmittel hat, die an gegenüberliegenden Paneelkanten vorgesehen sind, wobei eine Paneelkante eine Kantenbrechung aufweist und die komplementäre Paneelkante ebenfalls eine Kantenbrechung hat, welche mit der vorgenannten Kantenbrechung gemeinsam eine V-Fuge bildet. Die Stoßfuge unter der V-Fuge ist relativ zur Paneeloberfläche geneigt und weist einen Spalt auf. Eine der Stoßflächen ist einer kleinen keilförmigen Feder zugeordnet, die an der oberen Kante des Paneels angeordnet ist und sie ist in Richtung ihres freien Endes abwärts geneigt. Die komplementäre obere Stoßfläche ist einer kleinen keilförmigen Nut zugeordnet, die an der oberen Kante des komplementären Paneels liegt. Diese obere Stoßfläche ist in Richtung des Grundes der betreffenden Nut abwärts geneigt. Die keilförmige Feder sowie die keilförmige Nut tragen bei diesem Stand der Technik, wenn überhaupt, nur unwesentlich zur vertikalen Verriegelung bei; stattdessen ist zwecks einer mit V1 vermerkten vertikalen Verriegelung eigens an anderer Stelle eine Feder und Nut vorgesehen. Bei diesem Stand der Technik soll eine Verriegelung geschaffen werden, welche eine relative Position der Paneele erzeugen kann, die als anfänglich gegenseitig verriegelnde Position bezeichnet wird. Aus dieser Position heraus sollen die Paneele dann in horizontaler Richtung aufeinander zu oder voneinander weg bewegbar sein. In beiden Richtungen soll eine rückstellende Kraft erzeugbar sein, nämlich rückstellend in Richtung der anfänglich gegenseitig verriegelnden Position. Die Rückstellwirkung soll auf einer elastischen Biegung der unteren Nutwand beruhen. Darüber hinaus soll die Verriegelung in horizontaler Richtung frei von Spiel sein, wobei eine horizontale Position, bei der die untere Nutwand elastisch nach unten gebogen ist, im Sinne dieses Standes der Technik als spielfrei bezeichnet wird.

Nachteilig ist der obige Stand der Technik deshalb, weil die Paneeloberfläche desjenigen Paneels mit der zwecks vertikaler Verriegelung vorgesehenen Feder unter dasjenige Niveau absinken kann, welches dessen Paneeloberfläche hatte, als die anfänglich gegenseitig verriegelnden Position eingenommen wurde. Die Gestaltung begünstigt einen Höhenversatz zwischen den verriegelten Paneelkanten, Verschleiß der Verriegelungsmittel wird verstärkt und der Zusammenhalt verriegelter Paneelkanten wird geschwächt. Der Erfindung liegt die Aufgabe zugrunde, ein Paneel vorzuschlagen, dessen Verriegelungsmittel besser zusammenhalten, wenn verriegelte Paneelkanten voneinander weg bewegt werden.

Erfindungsgemäß wird die Aufgabe dadurch gelöst, dass die komplementären Halteprofile zweckmäßig so gestaltet sind, dass sie sich durch eine Schwenkbewegung formschlüssig verbinden lassen, mit der Maßgabe, dass an der Paneeloberfläche zweier verbundener Paneelkanten eine geschlossene Stoßfuge erzeugbar ist, mit der Maßgabe, dass durch die beiden Stoßflächen im Kontakt miteinander ein erstes Bereichsende eines Bewegungsspielraums definiert ist, und zwar für eine Bewegung der Paneele relativ zueinander und in einer Bewegungsrichtung, die sowohl senkrecht zu den formschlüssig verbundenen Paneelkanten als auch parallel zur Ebene der verbundenen Paneele liegt, wobei jedes der Halteprofile je eine untere Anschlagfläche aufweist, welche dann maximal voneinander beabstandet sind, wenn die oberen Stoßflächen einander berühren und die Stoßfuge geschlossen ist, und wobei der maximale Abstand zwischen den unteren Anschlagflächen die Größe des Bewegungsspielraums bemisst, und wobei die unteren Anschlagflächen, wenn sie einander berühren, das zweite Bereichsende des Bewegungsspielraums definieren und wobei eine Federunterseite der Verriegelungsfeder eine untere Kontaktfläche aufweist und die untere Nutwand der Verriegelungsnut mit einer Auflagefläche für die untere Kontaktfläche der Verriegelungsfeder versehen ist, wobei die untere Kontaktfläche der Verriegelungsfeder parallel zur Paneeloberfläche angeordnet ist und sich die Auflagefläche ebenfalls parallel zur Paneeloberfläche erstreckt, und wobei die Auflagefläche für die untere Kontaktfläche in Gebrauchslage horizontal angeordnet ist.

In einer einfachen Ausführungsform ist die betreffende Feder, an welcher die obere Stoßfläche vorgesehen ist, durch die Verriegelungsfeder gebildet und die betreffende Nut, welche mit der oberen Stoßfläche versehen ist, ist durch die Verriegelungsnut gebildet. Außerdem ist dann die untere Anschlagfläche des einen Halteprofils an der Federunterseite der Verriegelungsfeder vorgesehen und die Anschlagfläche des komplementären Halteprofils ist an der unteren Nutwand der Verriegelungsnut vorgesehen.

Wenn zwei Paneele miteinander verriegelt sind, dann verlaufen die einander gegenüberliegenden Stoßflächen mit einer Neigung gegenüber dem Lot auf der Paneeloberfläche. Die oberen Stoßflächen sind vorzugsweise parallel zueinander angeordnet. Durch die parallele Anordnung können sich die Stoßflächen dann flächig berühren, wenn die Stoßfuge geschlossen ist. Die Stoßflächen schmiegen sich dann aneinander. Wenn sich zwei verriegelte Paneele voneinander weg bewegen und sich zwischen den Stoßflächen ein Spalt bildet, dann ist die Weite dieses Spaltes (Spaltmaß) stets geringer als der zugehörige horizontale Verschiebeweg, um den sich die Paneele auseinander bewegt haben. Trigonometrisch kann der Verschiebeweg der Paneele als Hypotenuse eines rechtwinkligen Dreiecks aufgefasst werden, während das Spaltmaß dann einer der Katheten dieses Dreiecks entspricht. Vorteilhafterweise stört ein verengter Spalt das Erscheinungsbild eines Fußbodenbelags in geringerem Maß als beim Stand der Technik. Ein weiterer Vorteil ist, dass eine der beiden Stoßflächen für einen Betrachter stets verdeckt und überhaupt nicht sichtbar ist. Die andere Stoßfläche erlaubt wegen ihrer Neigung gegenüber dem Lot auf der Paneeloberfläche günstigerweise keinen tiefen Blick in den Spalt. Je geringer die Neigung, umso geringer ist die mögliche Blicktiefe in den Spalt.

Darüber hinaus hat ein verengter Spalt einen weiteren Vorteil, weil nämlich Schmutz schlechter eindringen kann als in einen Spalt, der eine größere Weite aufweist.

Die vorliegende Erfindung ist für Paneele mit einer Trägerplatte beziehungsweise einem Rumpf aus Holz oder aus weiterverarbeiteten Holzwerkstoffen vorgesehen, aber auch für Paneele mit einem Rumpf aus Kunststoff oder aus einem Holz-Kunststoff-Komposit-Werkstoff, engl. Wood Particle Composite (WPC). In Komposit-Werkstoffen kann Holz auch durch organische und/oder mineralische Füllstoffe ersetzt oder um diese Werkstoffe ergänzt sein, beispielsweise Steinmehl, Asche, Ruß, zerkleinerte pflanzliche Bestandteile, wie Reisschrot, Bambus-, Korkbestandteile, etc. Der Aufbau der Trägerplatten aus Komposit-Werkstoff oder solche aus Holz-Kunststoff-Komposit-Werkstoff kann mehrere Schichten umfassen, die unterschiedliche Zusammensetzung und unterschiedliche Eigenschaften aufweisen, beispielsweise eine elastische Schicht mit Dämpfungseigenschaften oder eine Diffusions- oder Sperrschicht, welche die Durchlässigkeit für Feuchte beeinflussen, etc.

Wenn eine Trägerplatte aus einem Komposit-Werkstoff oder aus einem Holz-Kunststoff-Komposit-Werkstoff hergestellt ist, und aus dieser Trägerplatte ein Paneel produziert wurde, dann kann am Beginn des Lebenszyklus des Paneels ein Wachstum auftreten, das durch eine gewisse Aufnahme von Feuchtigkeit bedingt ist. Die maximale Aufnahmefähigkeit für Feuchtigkeit ist aber begrenzt und je nach Zusammensetzung der Trägerplatte unterschiedlich. Sobald das Paneel eine gewisse Sättigung mit Feuchtigkeit erreicht hat, stellt sich ein Wachstumszustand ein, der anschließend nur noch in kleineren Grenzen variiert und zwar dann im Wesentlichen aufgrund von klimatischen Veränderungen. Sowohl Änderungen der Umgebungstemperatur als auch der Luftfeuchtigkeit in der unmittelbaren Umgebung beeinflussen die Größe des Paneels und können eine gewisse Schrumpfung oder eine gewisse Ausdehnung hervorrufen.

Wenn ein Fußbodenbelag aus solchen Paneelen zusammengesetzt ist, ergibt dies eine Fußbodenscheibe. Eine gewisse Schrumpfung beziehungsweise Ausdehnung der einzelnen Paneele kumuliert und führt zu einer Schrumpfung beziehungsweise. Ausdehnung der Fußbodenscheibe insgesamt. Günstig ist der Bewegungsspielraum innerhalb verriegelter Paneelkanten so bemessen, dass idealerweise alle Paneele um das maximale Maß schrumpfen können, ohne wesentliche Zugkräfte in die Nachbarpaneele zu übertragen, und ebenso idealerweise könne sich alle Paneele um das maximale Maß ausdehnen, ohne wesentliche Druckkräfte in die Nachbarpaneele zu übertragen. Für jedes Paneel ist also ausreichend Platz geschaffen, um sich in dem Maße auszudehnen beziehungswiese schrumpfen zu können, wie es benötigt wird, um einerseits eine Aufwerfung des Fußbodenbelags zu vermeiden beziehungsweise andererseits, um eine Spaltbildung zu vermeiden, die das maximale Maß eines Spaltes B überschreiten, und die Paneelkanten beschädigen würde.

*Wenn der Rumpf zumindest teilweise aus einem Kunststoff besteht, dann kann eine Ausgestaltung aus einem Rumpf aus einem Kunststoff oder aus einem Holz-Kunststoff-Komposit-Werkstoff (WPC) bestehen. Die Trägerplatte beziehungsweise der Rumpf ist beispielsweise aus einem thermoplastischen, elastomeren oder duroplastischen Kunststoff ausgebildet. Des Weiteren sind Recyclingwerkstoffe aus den genannten Materialien im Rahmen der Erfindung einsetzbar. Bevorzugt wird dabei Plattenmaterial eingesetzt, insbesondere aus thermoplastischem Kunststoff, wie Polyvinylchlorid, Polyolefine (beispielsweise Polyethylen (PE), Polypropylen (PP), Polyamide (PA), Polyurethane (PU), Polystyrol (PS), AcrylnitrilButadien-Styrol (ABS), Polymethylmethacrylat (PMMA), Polycarbonat (PC), Polyethylenterephthalat (PET), Polyetheretherketon (PEEK) oder Mischungen oder Co-Polymerisate. Dabei können unabhängig von dem Grundmaterial der Trägerplatte beispielsweise Weichmacher vorgesehen sein, die etwa in einem Bereich von ≥0 Gew.-% bis ≤20 Ges.-%, insbesondere ≤10 Ges.-%, vorzugsweise ≤7 Ges.-%, beispielsweise in einem Bereich von ≥5 Gew.-% bis ≤10 Gew.-% vorliegen können. Ein geeigneter Weichmacher umfasst etwa den unter der Handelsbezeichnung "Dinsch" von der Firma BASF vertriebenen Weichmacher. Ferner können als Ersatz für herkömmliche Weichmacher Copolymere, wie etwa Acrylate oder Methacrylate, vorgesehen sein.*

*Insbesondere thermoplastische Kunststoffe bieten auch den Vorteil, dass die aus ihnen hergestellten Produkte sehr leicht rezykliert werden können. Es können auch Recycling-Materialien aus anderen Quellen verwendet werden. Hierdurch ergibt sich eine weitere Möglichkeit zur Senkung der Herstellungskosten.*

*Derartige Trägerplatten sind dabei sehr elastisch beziehungsweise federnd, was einen komfortablen Eindruck beim Begehen erlaubt und ferner die auftretenden Geräusche bei einem Begehen im Vergleich zu herkömmlichen Materialien reduzieren kann, somit eine verbesserter Trittschalldämmung realisierbar sein kann.*

*Darüber hinaus bieten die vorgenannten Trägerplatten den Vorteil einer guten Wasserfestigkeit, da sie eine Quellung von 1% oder weniger aufweisen. Dies gilt in überraschender Weise neben reinen Kunststoffträgern auch für WPC-Werk-stoffe, wie diese nachfolgend im Detail erläutert sind.*

*In besonders vorteilhafter Weise kann das Trägermaterial Holz-Polymer-Werkstoffe (Wood Plastic Composite, WPC) aufweisen oder daraus bestehen. Hier kann beispielhaft ein Holz und ein Polymer geeignet sein, welches in einem Verhältnis von 40*/*60 bis 70*/*30, beispielsweise 50*/*50 vorliegen kann. Als polymere Bestandteile können etwa Polypropylen, Polyethylen oder ein Copolymer aus den beiden vorgenannten Materialien verwendet werden. Derartige Materialien bieten den Vorteil, dass diese bereits bei geringen Temperaturen, wie etwa in einem Bereich von ≥180 °C bis ≤200 °C in dem vorbeschriebenen Verfahren zu einer Trägerplatte geformt werden können, so dass eine besonders effektive Prozessführung, etwa mit beispielhaften Liniengeschwindigkeiten in einem Bereich von 6m*/*min, ermöglicht werden kann. Beispielsweise sind für ein WPC-Produkt mit einer 50*/*50 Verteilung der Holz- und Polymeranteile bei einer beispielhaften Produktstärke von 4,1 mm möglich, was einen besonders effektiven Herstellungsprozess ermöglichen kann.*

*Ferner können so sehr stabile Paneele erzeugt werden, die weiterhin eine hohe Elastizität aufweisen, was insbesondere für eine effektive und kostengünstige Ausgestaltung von Verbindungselementen an dem Randbereich der Trägerplatte und ferner bezüglich einer Trittschalldämmung von Vorteil sein kann. Ferner kann auch die vorgenannte gute Wasserverträglichkeit mit einer Quellung von unter 1% bei derartigen WPC-Materialien ermöglicht werden. Dabei können WPC-Werkstoffe beispielsweise Stabilisatoren und*/*oder andere Additive aufweisen, welche bevorzugt im Kunststoffanteil vorliegen können.*

*Weiterhin kann es besonders vorteilhaft sein, dass die Trägerplatte ein PVC-basiertes Material umfasst oder daraus besteht. Auch derartige Materialien können in besonders vorteilhafter Weise für hochwertige Paneele dienen, welche etwa auch in Feuchträumen problemlos verwendbar sind. Ferner bieten sich auch PVC-basierte Materialien für die Trägerplatte für einen besonders effektiven Herstellungsprozess an, da hier etwa Liniengeschwindigkeiten von 8m*/*min bei einer beispielhaften Produktstärke von 4,1 mm möglich sein können, was einen besonders effektiven Herstellungsprozess ermöglichen kann. Ferner weisen auch derartige Trägerplatten eine vorteilhafte Elastizität und Wasserverträglichkeit auf, was zu den vorgenannten Vorteilen führen kann.*

*Bei Kunststoff-basierten Paneelen wie auch bei WPC-basierten Paneelen können dabei mineralische Füllstoffe von Vorteil sein. Besonders geeignet sind hier etwa Talk oder auch Kalziumcarbonat (Kreide), Aluminiumoxid, Kieselgel, Quarzmehl, Holzmehl, Gips. Beispielsweise kann Kreide vorgesehen sein in einem Bereich von ≥30 Gew.*-*% bis ≤70 Ges.-%, wobei durch die Füllstoffe, insbesondere durch die Kreide insbesondere der Schlupf der Trägerplatte verbessert werden kann. Auch können sie in bekannter Weise eingefärbt sein. Insbesondere kann es vorgesehen sein, dass das Material der Trägerplatten ein Flammschutzmittel aufweist.*

*Gemäß einer besonders bevorzugten Ausgestaltung der Erfindung besteht das Material der Trägerplatte aus einer Mischung eines PE*/*PP Blockcopolymers mit Holz. Dabei kann der Anteil des PE*/*PP Blockcopolymers sowie der Anteil des Holzes zwischen ≥45 Ges.-% und ≤55 Ges.-% liegen. Des Weiteren kann das Material der Trägerplatte zwischen ≥0 Ges.-% und ≤10 Gew.-% weiterer Additive, wie beispielsweise Fließhilfsmittel, Thermostabilisatoren oder UV-Stabilisatoren, aufweisen. Die Partikelgröße des Holzes liegt dabei zwischen >0 µm und ≤600 µm mit einer bevorzugten Partikelgrößenverteilung D50 von ≥400 µm. Insbesondere kann das Material der Trägerplatte dabei Holz mit einer Partikelgrößenverteilung D10 von ≥400 µm aufweisen. Die Partikelgrößenverteilung ist dabei auf den volumetrischen Durchmesser bezogen und bezieht sich auf das Volumen der Partikel. Besonders bevorzugt wird dabei das Material der Trägerplatte als granulierte oder pelletierte vorextrudierte Mischung aus einem PE*/*PP Blockcopolymer mit Holzpartikeln der angegeben Partikelgrößenverteilung bereitgestellt. Das Granulat und*/*oder die Pellets können dabei bevorzugt etwa eine Korngröße in einem Bereich von ≥400 µm bis ≤10 mm, bevorzugt ≥600 µm bis ≤10 mm aufweisen, insbesondere ≥800 µm bis ≤10 mm.*

*Gemäß einer weiteren bevorzugten Ausgestaltung der Erfindung besteht die Trägerplatte aus einer Mischung eines PE*/*PP Polymerblends mit Holz. Dabei kann der Anteil des PE*/*PP Polymerblends sowie der Anteil des Holzes zwischen ≥45 Gew*.-% *und ≤55 Gew*.-% *liegen. Des Weiteren kann das Material der Trägerplatte zwischen ≥0 Ges.-% und ≤10 Gew.-% weiterer Additive, wie beispielsweise Fließhilfsmittel, Thermostabilisatoren oder UV-Stabilisatoren, aufweisen. Die Partikelgröße des Holzes liegt dabei zwischen >0 µm und ≤600 µm mit einer bevorzugten Partikelgrößenverteilung D50 von ≥400 µm. Insbesondere kann die Trägerplatte Holz mit einer Partikelgrößenverteilung D10 von ≥400 µm aufweisen. Die Partikelgrößenverteilung ist dabei auf den volumetrischen Durchmesser bezogen und bezieht sich auf das Volumen der Partikel. Besonders bevorzugt wird dabei das Material der Träger als granulierte oder pelletierte vorextrudierte Mischung aus einem PE*/*PP Polymerblend mit Holzpartikeln der angegeben Partikelgrößenverteilung bereitgestellt. Das Granulat und*/ *oder die Pellets können dabei bevorzugt etwa eine Korngröße in einem Bereich von ≥400 µm bis ≤10 mm, bevorzugt ≥600 µm bis ≤10 mm aufweisen, insbesondere ≥800 µm bis ≤10 mm.*

*In einer weiteren Ausgestaltung der Erfindung besteht das Material der Trägerplatte aus einer Mischung eines PP-Homo-polymers mit Holz. Dabei kann der Anteil des PP-Homopolymers sowie der Holzanteil zwischen ≥45 Gew.-% und ≤55 Gew.-% liegen. Des Weiteren kann das Material der Trägerplatte zwischen ≥0 Gew.-% und ≤10 Gew.-% weiterer Additive, wie beispielsweise Fließhilfsmittel, Thermostabilisatoren oder UV-Stabilisatoren, aufweisen. Die Partikelgröße des Holzes liegt dabei zwischen >0 µm und ≤600 µm mit einer bevorzugten Partikelgrößenverteilung D50 von ≥400 µm. Insbesondere kann die Trägerplatte dabei Holz mit einer Partikelgrößenverteilung D10 von ≥400 µm aufweisen. Die Partikelgrößenverteilung ist dabei auf den volumetrischen Durchmesser bezogen und bezieht sich auf das Volumen der Partikel. Besonders bevorzugt wird dabei das Material der Trägerplatte als granulierte oder pelletierte vorextrudierte Mischung aus einem PP-Homopolymer mit Holzpartikeln der angegeben Partikelgrößenverteilung bereitgestellt. Das Granulat und*/*oder die Pellets können dabei bevorzugt etwa eine Korngröße in einem Bereich von ≥400 µm bis ≤10 mm, bevorzugt ≥600 µm bis ≤10 mm aufweisen, insbesondere ≥800 µm bis ≤10 mm. In einer weiteren Ausgestaltung der Erfindung besteht das Material der Trägerplatte aus einer Mischung eines PVC-polymers mit Kreide. Dabei kann der Anteil des PVC-Polymers sowie der Kreideanteil zwischen ≥45 Gew.-% und ≤55 Gew.-% liegen. Des Weiteren kann das Material der Trägerplatte zwischen ≥0 Ges.-% und ≤10 Gew.-% weiterer Additive, wie beispielsweise Fließhilfsmittel, Thermostabilisatoren oder UV-Stabilisatoren, aufweisen. Die Partikelgröße der Kreide liegt dabei zwischen ≥0 µm und ≤600 µm mit einer bevorzugten Partikelgrößenverteilung D50 von ≥400 µm. Insbesondere kann das Material der Trägerplatte dabei Kreide mit einer Partikelgrößenverteilung D10 von ≥400 µm aufweisen. Die Partikelgrößenverteilung ist dabei auf den volumetrischen Durchmesser bezogen und bezieht sich auf das Volumen der Partikel. Besonders bevorzugt wird dabei das Material der Trägerplatte als granulierte oder pelletierte vorextrudierte Mischung aus einem PVC-Polymer mit Kreide der angegeben Partikelgrößenverteilung bereitgestellt. Das Granulat und*/*oder die Pellets können dabei bevorzugt etwa eine Korngröße in einem Bereich von ≥400 µm bis ≤10 mm, bevorzugt ≥600 µm bis ≤10 mm aufweisen, insbesondere ≥800 µm bis ≤10 mm.*

*In einer weiteren Ausgestaltung der Erfindung besteht das Material der Trägerplatte aus einer Mischung eines PVC-Polymers mit Holz. Dabei kann der Anteil des PVC-Polymers sowie der Holzanteil zwischen ≥45 Gew.-% und ≤55 Gew.-% liegen. Des Weiteren kann das Material der Trägerplatte zwischen ≥0 Ges.-% und ≤10 Gew.-% weiterer Additive, wie beispielsweise Fließhilfsmittel, Thermostabilisatoren oder UV-Stabilisatoren, aufweisen. Die Partikelgröße des Holzes liegt dabei zwischen >0 µm und ≤600 µm mit einer bevorzugten Partikelgrößenverteilung D50 von ≥400 µm. Insbesondere kann das Material der Trägerplatte Holz mit einer Partikelgrößenverteilung D10 von ≥400 µm aufweisen. Die Partikelgrößenverteilung ist dabei auf den volumetrischen Durchmesser bezogen und bezieht sich auf das Volumen der Partikel. Besonders bevorzugt wird dabei das Material der Trägerplatte als granulier*te *oder pelletierte vorextrudierte Mischung aus einem PVC-Polymer mit Holzpartikeln der angegeben Partikelgrößenverteilung bereitgestellt. Das Granulat und*/*oder die Pellets können dabei bevorzugt etwa eine Korngröße in einem Bereich von ≥400 µm bis ≤10 mm, bevorzugt ≥600 µm bis ≤10 mm aufweisen, insbesondere ≥800 µm bis ≤10 mm.*

*Zur Bestimmung der Partikelgrößenverteilung kann auf die allgemein bekannten Verfahren, wie beispielsweise die Laserdiffraktometrie, zurückgegriffen werden, mit diesem Verfahren können Partikelgrößen im Bereich von einigen Nanometern bis hin zu mehreren Millimetern bestimmt werden. Es lassen sich damit auch D50 bzw. D10 Werte ermitteln, welche 50*% *bzw. 10% der gemessenen Partikel kleiner sind als der angegebene Wert.*

*Gemäß einer weiteren Ausgestaltung der Erfindung weist das Trägermaterial ein einen Kunststoff aufweisendes Matrixmaterial und ein Feststoffmaterial auf, wobei das Feststoffmaterial zu wenigstens 50 Gew.-%, insbesondere zu wenigstens 80 Gew.-%, besonders bevorzugt zu wenigstens 95 Gew.-%, bezogen auf das Feststoffmaterial, durch Talkum gebildet ist. Dabei liegt das Matrixmaterial in einer Menge, bezogen auf das Trägermaterial, von ≥ 30 Gew.-% bis ≤ 70 Gew.-%, insbesondere von ≥ 40 Gew.-% bis ≤ 60 Gew.-%, vor und liegt das Feststoffmaterial, bezogen auf das Trägermaterial, in einer Menge, bezogen auf das Trägermaterial, von ≥ 30 Gew.-% bis ≤* 70 *Gew*.-%, *insbesondere von ≥ 40 Gew*.-% *bis ≤ 60 Gew*.-%, *beispielsweise kleiner oder gleich 50 Gew.-% vor. Weiterhin ist es vorgesehen, dass das Trägermaterial und das Feststoffmaterial gemeinsam, bezogen auf das Trägermaterial in einer Menge von ≥ 95 Gew.-%, insbesondere ≥ 99 Gew.-%, vorliegen.*

*Das Feststoffmaterial kann in einer solchen Ausgestaltung der Erfindung zu wenigstens 50 Gew.-%, insbesondere zu wenigstens 80 Gew.-%, beispielsweise zu 100%, bezogen auf das Feststoffmaterial, durch Talkum gebildet sein. Unter Talkum wird dabei in an sich bekannter Weise ein Magnesiumsilikathydrat verstanden, welches beispielsweise die chemische Summenformel Mg3 [Si4010 (OH) 2] aufweisen kann. Somit ist der Feststoffanteil vorteilhafter Weise zumindest durch einen Großteil aus dem mineralischen Stoff Talkum gebildet, wobei dieser Stoff etwa als Pulverform eingesetzt werden kann beziehungsweise in dem Trägermaterial in Form von Partikeln vorliegen kann. Grundsätzlich kann das Feststoffmaterial aus einem pulverförmigen Feststoff bestehen.*

*Vorteilhaft kann es sein, wenn die spezifische Oberflächendichte nach BET, ISO 4652 der Talkum-Partikel in einem Bereich liegt von ≥ 4 m2*/*g bis ≤ 8 m2*/*g, etwa in einem Bereich von ≥ 5 m2*/*g bis ≤* 7 *m2*/*g.*

*Weiterhin kann es vorteilhaft sein, wenn das Talkum bei einer Schüttdichte nach DIN 53468 vorliegt in einem Bereich von ≥ 0,15 g*/*cm3 bis ≤ 0,45 g*/*cm3, etwa in einem Bereich von ≥ 0,25 g*/*cm3 bis ≤ 0,35 g*/*cm3.*

*Das Matrixmaterial in einer solchen Ausgestaltung der Erfindung dient insbesondere dazu, bei dem fertig hergestellten Träger das Feststoffmaterial aufzunehmen beziehungsweise einzubetten. Das Matrixmaterial weist dabei einen Kunststoff oder eine Kunststoffmischung auf. Insbesondere mit Bezug auf das Herstellungsverfahren, wie dies nachfolgend im Detail beschrieben ist, kann es vorteilhaft sein, dass das Matrixmaterial einen thermoplastischen Kunststoff aufweist. Dadurch wird es ermöglicht, dass das Trägermaterial beziehungsweise ein Bestandteil des Trägermaterials einen Schmelzpunkt oder einen Erweichungspunkt aufweist, um das Trägermaterial in einem weiteren Verfahrensschritt durch Hitzeeinwirkung zu Formen, wie dies nachstehend mit Bezug auf das Verfahren im Detail beschrieben ist. Das Matrixmaterial kann insbesondere aus einem Kunststoff beziehungsweise einem Kunststoffgemisch und gegebenenfalls einem Haftvermittler bestehen. Bevorzugt können diese Komponenten zumindest 90Gew.-%, besonders bevorzugt zumindest 95 Ges.-%, insbesondere wenigstens 99 Gew*.-% *des Matrixmaterials ausmachen.*

*Ferner kann es vorgesehen sein, dass das Matrixmaterial in einer Menge, bezogen auf das Trägermaterial, von ≥ 30 Gew.-% bis ≤* 70 *Ges.-%, insbesondere von ≥ 40 Gew.-% bis ≤ 60 Gew.-% vorliegt. Weiterhin ist es vorgesehen, dass das Feststoffmaterial, bezogen auf das Trägermaterial, in einer Menge, bezogen auf das Trägermaterial, von ≥ 30 Ges.-% bis ≤* 70 *Gew.-%, insbesondere von ≥ 40 Gew*.-% *bis ≤ 60 Gew.-%, vorliegt.*

*Polypropylen ist als Matrixmaterial besonders geeignet, da es zum einen kostengünstig erhältlich ist und ferner als thermoplastischer Kunststoff gute Eigenschaften als Matrixmaterial zum Einbetten des Feststoffmaterials aufweist. Dabei kann insbesondere eine Mischung aus einem Homopolymer und einem Copolymer für das Matrixmaterial besonders vorteilhafte Eigenschaften ermöglichen. Derartige Materialien bieten ferner den Vorteil, dass diese bereits bei geringen Temperaturen, wie etwa in einem Bereich von ≥ 180 °C bis ≤ 200 °C in dem vorbeschriebenen Verfahren zu einem Träger geformt werden können, so dass eine besonders effektive Prozessführung, etwa mit beispielhaften Liniengeschwindigkeiten in einem Bereich von 6m*/*min, ermöglicht werden kann.*

*Weiterhin kann es vorteilhaft sein, wenn das Homopolymer eine Zugfestigkeit nach ISO* 527-2 *aufweist, die in einem Bereich liegt von ≥ 30 MPa bis ≤ 45 MPa, beispielsweise in einem Bereich von ≥ 35 MPa bis ≤ 40 MPa, um eine gute Stabilität zu erreichen.*

*Ferner kann insbesondere für eine gute Stabilität es von Vorteil sein, wenn das Homopolymer ein Biegemodul nach ISO 178 aufweist in einem Bereich von ≥ 1000 MPa bis ≤ 2200 MPa, beispielsweise in einem Bereich von ≥ 1300 MPa bis ≤ 1900 MPa, etwa in einem Bereich von ≥ 1500 MPa bis ≤ 1700 MPa.*

*Bezüglich der Zugverformung des Homopolymers nach ISO 527-2 kann es ferner von Vorteil sein, wenn diese in einem Bereich liegt von ≥ 5% bis ≤ 13%, beispielsweise in einem Bereich von ≥ 8*% *MPa bis ≤ 10*%.

*Für eine besonders vorteilhafte Herstellbarkeit kann es vorgesehen sein, dass die Vicat-Erweichungstemperatur nach ISO 306*/*A für ein spritzgegossenes Bauteil, in einem Bereich liegt von ≥ 130 °C MPa bis ≤ 170 °C, beispielsweise in einem Bereich von ≥ 145 °C bis ≤ 158 °C.*

*Es kann weiterhin vorteilhaft sein, dass das Feststoffmaterial neben Talkum wenigstens einen weiteren Feststoff aufweist. Diese Ausgestaltung kann es insbesondere ermöglichen, dass das Gewicht des Trägermaterials beziehungsweise eines mit dem Trägermaterial ausgebildeten Paneels verglichen mit einem Trägermaterial beziehungsweise Paneel, bei dem das Feststoffmaterial aus Talkum besteht, deutlich reduziert sein kann. Somit kann der dem Feststoffmaterial zugesetzte Feststoff insbesondere eine verglichen mit Talkum reduzierte Dichte aufweisen. Beispielsweise kann der zugesetzte Stoff eine Rohdichte aufweisen, die in einem Bereich liegt von ≤ 2000 kg*/*m3, insbesondere von ≤ 1500 kg*/*m3, beispielsweise von ≤ 1000 kg*/*m3, besondere bevorzugt von ≤ 500 kg*/*m3. In Abhängigkeit des zugesetzten Feststoffs kann dabei ferner eine weitere Adaptierbarkeit an die gewünschten insbesondere mechanischen Eigenschaften ermöglicht werden.*

*Beispielhaft kann der weitere Feststoff ausgewählt sein aus der Gruppe bestehend aus Holz, etwa in Form von Holzmehl, Blähton Vulkanasche, Bims, Porenbeton, insbesondere anorganischen Schäumen, Cellulose. Mit Bezug auf Porenbeton kann dies beispielsweise der von der Firma Xella unter dem Markennamen YTONG verwendete Feststoff sein, der im Wesentlichen aus Quarzsand, Kalk und Zement besteht, beziehungsweise kann der Porenbeton die vorgenannten Bestandteile aufweisen. Mit Bezug auf den zugesetzten Feststoff kann dieser Beispielsweise aus Partikeln aufgebaut sein, die die gleiche Partikelgröße beziehungsweise Partikelgrößenverteilung aufweisen, wie die vorstehend für Talkum vorbeschriebenen Partikelgrößen beziehungsweise Partikelgrößenverteilungen. Die weiteren Feststoffe können insbesondere in einem Anteil in dem Feststoffmaterial vorliegen, der in einem Bereich von < 50 Gew*.-%, *insbesondere < 20 Gew.-%, beispielsweise < 10 Ges.-%, weiter beispielsweise < 5 Ges.-%, liegt.*

*Alternativ kann es beispielsweise für Holz, insbesondere für Holzmehl vorgesehen sein, dass dessen Partikelgröße zwischen >0µm und ≤600µm mit einer bevorzugten Partikelgrößenverteilung D50 von ≥400µm liegt.*

*Gemäß einer weiteren Ausgestaltung kann das Material der Trägerplatte Mikrohohlkugeln aufweisen. Derartige Zusatzstoffe können insbesondere bewirken, dass die Dichte der Trägerplatte und damit des erzeugten Paneels signifikant reduziert werden kann, so dass ein besonders einfacher und kostengünstiger Transport und ferner ein besonders komfortables Verlegen gewährleistet werden kann. Dabei kann insbesondere durch das Einfügen von Mikrohohlkugeln eine Stabilität des erzeugten Paneels gewährleistet werden, welche im Vergleich zu einem Material ohne Mikrohohlkugeln nicht signifikant reduziert ist. Somit ist die Stabilität für einen Großteil der Anwendungen vollkommen ausreichend. Unter Mik-rohohlkugeln können dabei insbesondere Gebilde verstanden werden, welche einen hohlen Grundkörper aufweisen und eine Größe beziehungsweise einen maximalen Durchmesser aufweisen, der im Mikrometerbereich liegt. Beispielsweise können verwendbare Hohlkugeln einen Durchmesser aufweisen, welcher im Bereich von ≥5 µm bis ≤100 µm, beispielsweise ≥20 µm bis ≤50 µm liegt. Als Material der Mikrohohlkugeln kommt grundsätzlich jegliches Material in Betracht, wie beispielsweise Glas oder Keramik. Ferner können aufgrund des Gewichts Kunststoffe, etwa die auch in dem Trägermaterial verwendeten Kunststoffe, beispielsweise PVC, PE oder PP, vorteilhaft sein, wobei diese gegebenenfalls, etwa durch geeignete Zusatzstoffe, an einem Verformen während des Herstellungsverfahrens gehindert werden können.*

*Die Härte des Materials der Trägerplatte kann Werte in einem Bereich von 30-90 N*/*mm² (gemessen nach Brinell) aufweisen. Der E-Modul kann in einem Bereich von 3.000 bis 7.000 N*/*mm² liegen.*

Die Erfindung ist vorzugsweise vorgesehen für Paneele mit einer Gesamtdicke, die 2 mm und mehr beträgt. Für Paneele, deren Gesamtdicke unter 4 mm liegt, werden bevorzugt Trägerplatten mit überwiegendem Kunststoffanteil verwendet.

Eine Weiterbildung sieht vor, dass an dem Rand des Halteprofils, das die Verriegelungsfeder aufweist, eine Kantenbrechung vorgesehen ist, dass die Kantenbrechung als Fase ausgebildet ist, dass die Fase zum freien Ende der Verriegelungsfeder hin abwärts geneigt ist, und dass die obere Stoßfläche dieses Halteprofils ebenfalls zum freien Ende der Verriegelungsfeder hin abwärts geneigt ist, wobei der Neigungswinkel der oberen Stoßfläche kleiner oder gleich groß oder größer als der Neigungswinkel der Fase ist.

Bei dieser Ausführungsform sind an der Paneelkante, welche mit der Verriegelungsfeder versehen ist, die Fase und die obere Stoßfläche in dieselbe Richtung abwärts geneigt. Dieses Prinzip wird jedoch bei anderen Ausführungsformen durchbrochen und zwar bei solchen, die an einem Halteprofil zusätzlich zu der Verriegelungsfeder auch eine Verschlussnut aufweisen, weil dann die obere Stoßfläche der Verschlussnut zugeordnet ist und deswegen eine gegensätzliche Neigung aufweisen muss, wie weiter unten dargelegt.

Besonders einfach gestaltet sich das Paneel, wenn die Federoberseite der Verriegelungsfeder in Bezug zur Paneeloberfläche geneigt ist und die Federoberseite und die obere Stoßfläche zu einer gemeinsamen Fläche integriert sind, wobei die Verriegelungsnut an der Innenseite ihrer oberen Nutwand ebenfalls in Bezug zur Paneeloberfläche geneigt ist, und wobei deren Neigung angepasst ist an die Neigung der oberen Stoßfläche der Verriegelungsfeder.

Die Federoberseite und die Stoßfläche sind zu einer Fläche integriert. Dadurch wird auf eine Kerbstelle beziehungsweise einen Knickpunkt zwischen Federoberseite und Stoßfläche verzichtet. Weil eine Kerbstelle in der Kontur eingespart wird, weist das Halteprofil eine höhere Festigkeit auf. Außerdem ist die Kontur insgesamt einfacher gestaltet und eignet sich deswegen besser für sehr dünne Paneele. Je geringer die Gesamtdicke des Paneels ist, desto einfacher sollte das Halteprofil gestaltet sein, weil insbesondere eine Kontur mit sehr feinen Details schwieriger herzustellen ist, je dünner ein Paneel ist.

Ein weiterer Nutzen ergibt sich dadurch, dass die Verriegelungsnut eine untere Nutwand hat, an deren freiem Ende ein zur Paneeloberseite gerichteter Halterand vorgesehen ist, dass die untere Anschlagfläche der Verriegelungsnut an dem Halterand der unteren Nutwand angeordnet ist, und dass das Lot auf der Anschlagfläche nach innen zum Nutgrund der Verriegelungsnut gerichtet ist. Es hat sich erwiesen, dass eine Verriegelung mit unteren Anschlagflächen besonders haltbar und stabil ist, wenn sie von der Paneeloberfläche entfernt im Bereich einer unteren Nutwand realisiert ist.

Der Erfinder hat nach dem Grund dafür gesucht, warum eine elastische Biegung der unteren Nutwand der Verriegelungsnut die Stoßflächen verriegelter Paneele aufeinander zu zwingt und dabei herausgefunden, dass von den unteren Anschlagflächen zumindest die Anschlagfläche der Verriegelungsnut in Bezug zur Paneeloberfläche senkrecht angeordnet sein kann, um ein Aufeinanderzuzwingen der Paneele zu vermeiden.

Zweckmäßig weist eine Federunterseite der Verriegelungsfeder eine untere Kontaktfläche auf, und die untere Nutwand der Verriegelungsnut ist mit einer Auflagefläche für die untere Kontaktfläche der Verriegelungsfeder versehen, wobei die untere Kontaktfläche der Verriegelungsfeder parallel zur Paneeloberfläche angeordnet ist und sich die Auflagefläche ebenfalls parallel zur Paneeloberfläche erstreckt.

Durch diese Maßnahme ist die Auflagefläche für die untere Kontaktfläche in Gebrauchslage horizontal angeordnet. Die flache Kontaktfläche der Verriegelungsfeder kann dann von oben einwirkende Kräfte in die ebenfalls flache Auflagefläche der unteren Nutwand ableiten. Darüber hinaus dienen die Kontaktfläche und die Auflagefläche als Lagerung und Führung, wenn die Paneele im Rahmen des vorhandenen Bewegungsspielraums ihre relative Position zueinander verschieben.

Eine Weiterbildung des Paneels sieht vor, dass die Federunterseite der Verriegelungsfeder zumindest an einem der beiden Enden eine untere Kontaktfläche und einen ansteigenden Bereich aufweist.

Durch diese Gestaltung wird der Fügevorgang vereinfacht. Das Paneel mit der Verriegelungsfeder, das üblicherweise angewinkelt (schräg) und an die Verriegelungsnut eines liegenden Paneels angesetzt und dann mittels einer herabschwenkenden Bewegung der Verriegelungsfeder formschlüssig in die Verriegelungsnut eingefügt wird, lässt sich einfacher in die Verriegelungsnut eines liegenden Paneel einschwenken, wenn die Verriegelungsfeder wenigstens einen ansteigenden Bereich aufweist.

Eine Alternative sieht vor, dass das Halteprofil mit der Verriegelungsfeder oberhalb derselben eine Verschlussnut aufweist, und dass das komplementäre Halteprofil mit Verriegelungsnut mit seinem freien Ende der oberen Nutwand eine Verschlussfeder bildet, welche in die Verschlussnut einfügbar ist. Dadurch sind die für die Festigkeit der Verriegelung dienliche Verriegelungsfeder und die Verriegelungsnut von dem Ort entfernt und geschützt, an dem Schmutz unmittelbar eindringen kann. Die vorgeschaltete Verschlussfeder/ Verschlussnut halten Schmutz von der Verriegelungsfeder und die Verriegelungsnut fern.

Bei der vorgenannten Alternative kann die obere Stoßfläche derjenigen Paneelkante, welche mit der Verschlussfeder versehen ist, an der Federoberseite der Verschlussfeder angeordnet sein, und die obere Stoßfläche derjenigen Paneelkante, welche mit der Verschlussnut versehen ist, kann an der oberen Nutwand der Verschlussnut angeordnet sein.

Nachfolgend ist die Erfindung in einer Zeichnung beispielhaft veranschaulicht und anhand mehrerer Ausführungsbeispiele detailliert beschrieben. Es zeigen:
- Fig. 1a: ein erstes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 1b: das Ausführungsbeispiel gemäß Fig. 1a mit einander berührenden unteren Anlageflächen und einem Spalt an der maximal geöffneten Stoßfuge,
- Fig. 2a: ein zweites Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 2b: das Ausführungsbeispiel gemäß Fig. 2a mit einander berührenden unteren Anlageflächen und einem Spalt an der maximal geöffneten Stoßfuge,
- Fig. 3a: ein drittes Ausführungsbeispiel eines Paneels, wobei das Paneel zerteilt dargestellt ist, um dessen gegenüberliegende Paneelkanten im verriegelten Zustand mit geschlossener Stoßfuge darzustellen,
- Fig. 3b: das Ausführungsbeispiel gemäß Fig. 3a mit einander berührenden unteren Anlageflächen und einem Spalt an der maximal geöffneten Stoßfuge,
- Fig. 4a: ein viertes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 4b: das Ausführungsbeispiel gemäß Fig. 4a mit einander berührenden unteren Anlageflächen und einem Spalt an der maximal geöffneten Stoßfuge,
- Fig. 5a: ein fünftes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 5b: das Ausführungsbeispiel gemäß Fig. 5a mit einander berührenden unteren Anlageflächen und einem Spalt an der maximal geöffneten Stoßfuge,
- Fig. 6a: ein sechstes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 6b: das Ausführungsbeispiel gemäß Fig. 6a mit einander berührenden unteren Anlageflächen und einem Spalt an der maximal geöffneten Stoßfuge,
- Fig. 7a: ein siebentes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 7b: das Ausführungsbeispiel gemäß Fig. 7a mit einander berührenden unteren Anlageflächen und einem Spalt an der maximal geöffneten Stoßfuge,
- Fig. 7c: das Ausführungsbeispiel gemäß Fig. 7a/7b mit unteren Anlageflächen in einem gewissen Abstand voneinander sowie mit einem gewissen Spalt an der geöffneten Stoßfuge,
- Fig. 8a: ein achtes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 8b: das Ausführungsbeispiel gemäß Fig. 8a mit einander berührenden unteren Anlageflächen und einem Spalt an der maximal geöffneten Stoßfuge,
- Fig. 8c: das Ausführungsbeispiel gemäß Fig. 8a/8b mit unteren Anlageflächen in einem gewissen Abstand voneinander sowie mit einem gewissen Spalt an der geöffneten Stoßfuge,
- Fig. 9a: ein neuntes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 9b: das Ausführungsbeispiel gemäß Fig. 9a mit einander berührenden unteren Anlageflächen und einem Spalt an der maximal geöffneten Stoßfuge,
- Fig. 10a: ein zehntes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 10b: das Ausführungsbeispiel gemäß Fig. 10a mit einander berührenden unteren Anlageflächen und einem Spalt an der maximal geöffneten Stoßfuge,
- Fig. 10c: das Ausführungsbeispiel gemäß Fig. 10a/10b mit unteren Anlageflächen in einem gewissen Abstand voneinander sowie mit einem gewissen Spalt an der geöffneten Stoßfuge,
- Fig. 11a: ein elftes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 11b: das Ausführungsbeispiel gemäß Fig. 11a mit einander berührenden unteren Anlageflächen und einem Spalt an der maximal geöffneten Stoßfuge,
- Fig. 12a: ein zwölftes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 12b: das Ausführungsbeispiel gemäß Fig. 12a mit einander berührenden unteren Anlageflächen und einem Spalt an der maximal geöffneten Stoßfuge,
- Fig. 13a: ein dreizehntes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 13b: das Ausführungsbeispiel gemäß Fig. 13a mit einander berührenden unteren Anlageflächen und einem Spalt an der maximal geöffneten Stoßfuge,
- Fig. 14a: ein vierzehntes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 14b: das Ausführungsbeispiel gemäß Fig. 14a mit einander berührenden unteren Anlageflächen und einem Spalt an der maximal geöffneten Stoßfuge,
- Fig. 15a: ein vierzehntes Ausführungsbeispiel eines erfindungsgemäßen Paneels, wobei das Paneel zerteilt dargestellt ist, um dessen gegenüberliegende Paneelkanten im verriegelten Zustand mit geschlossener Stoßfuge darzustellen,
- Fig. 15b: das Ausführungsbeispiel gemäß Fig. 15a mit einander berührenden unteren Anlageflächen und einem Spalt an der maximal geöffneten Stoßfuge.

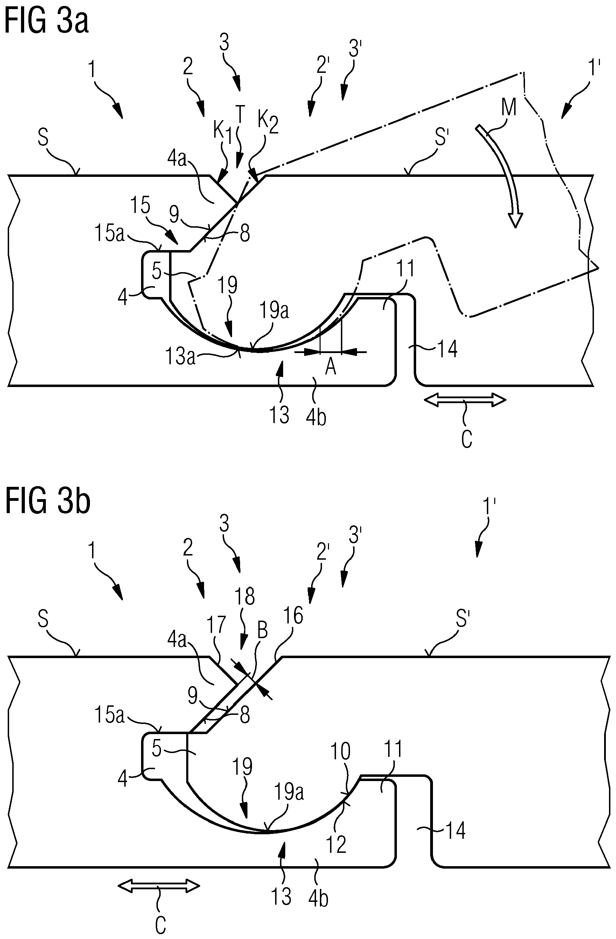

Die Ausführungsformen der Figuren 3a/3b, 5a/5b sowie 6a/6b fallen nicht unter den Schutz.

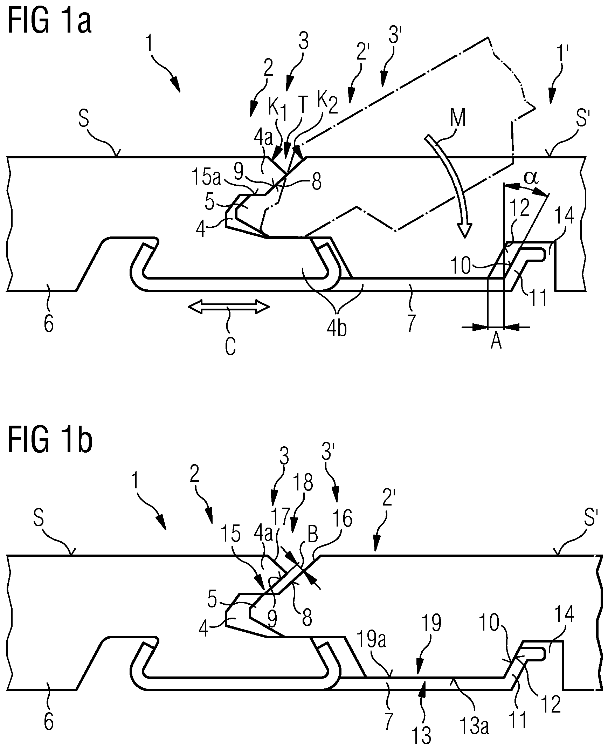

Fig. 1a zeigt ein erstes Ausführungsbeispiel eines erfindungsgemäßen Paneels. Das Paneel ist zerteilt dargestellt, um dessen gegenüberliegende Paneelkanten 1 und 1' im verriegelten Zustand mit geschlossener Stoßfuge darstellen zu können. Selbstverständlich können die ausschnittsweise dargestellten Paneelkanten auch als auschnittsweise Darstellung zweier Paneele aufgefasst werden, welche nicht durchtrennt sind.

In der Praxis ist es durchaus üblich, wenn beispielsweise ein Paneel am Ende einer Paneelreihe zu lang ist, dieses durchzutrennen, um es auf die benötigte Länge zu kürzen. Mit dem abgetrennten Reststück kann in der Regel eine neue Paneelreihe begonnen werden. Komplementäre Halteprofile eines durchtrennten Paneels passen ineinander und können im Prinzip miteinander verriegelt werden, wie in den vorliegenden Figurengruppen 1-15 dargestellt.

Das Paneel 1 beziehungsweise 1' der Figurengruppe 1 basiert auf einer klassischen Konstruktion der Firma Välinge Innovation AB, wie aus WO 1994/026999 bekannt. Es weist ein Paar komplementärer Verriegelungsmittel 2 und 2' an den dargestellten einander gegenüberliegenden Paneelkanten auf. Diese Verriegelungsmittel sind als formschlüssige Halteprofile 3 beziehungsweise 3' mit Verriegelungsnut 4 beziehungsweise mit komplementärer Verriegelungsfeder 5 ausgestaltet. Die Verriegelungsnut 4 umfasst eine oberen Nutwand 4a und eine untere Nutwand 4b. Dasjenige Halteprofil mit der Verriegelungsnut 4 ist zweistückig aufgebaut. Eines der beiden Bestandteile ist eine Paneelplatte 6. An deren Rand ist als zweiter Bestandteil ein separater Streifen 7 aus einem anderen Material formschlüssig fixiert. Der separate Streifen 7 bildet die unter Nutwand 4b der Verriegelungsnut 4.

Gegenüber dem klassischen Paneel ist es so modifiziert, dass die Paneeloberfläche S beziehungsweise S' an den Rändern beider Halteprofile 3 und 3' jeweils eine Kantenbrechung K₁ beziehungsweise K₂ aufweist. Unterhalb der Kantenbrechung ist jeweils eine obere Stoßfläche vorgesehen, nämlich sowohl an dem Halteprofil 3 mit Verriegelungsnut eine obere Stoßfläche 8, als auch an dem Halteprofil 3' mit der Verriegelungsfeder 5 eine obere Stoßfläche 9. In der in Fig. 1a dargestellten Position sind die Paneelkanten 1/1' so gegeneinander gestoßen, dass sich die beiden oberen Stoßflächen 8 und 9 berühren und eine geschlossene Stoßfuge T erzeugt ist. Die Stoßfuge T ist relativ zur Paneeloberfläche S beziehungsweise S' geneigt. Eine der oberen Stoßflächen 9 ist der Verriegelungsfeder 5 zugeordnet, und diese obere Stoßfläche 9 ist in Richtung des freien Endes der Verriegelungsfeder 5 abwärts geneigt. Die komplementäre obere Stoßfläche 8 ist der Verriegelungsnut 4 zugeordnet, und diese Stoßfläche 8 ist in Richtung des Nutgrundes der Verriegelungsnut 4 abwärts geneigt. Die komplementären Halteprofile 3 und 3' sind so gestaltet, dass sie sich durch eine Schwenkbewegung M des einen Paneels 1', das mit seiner Verriegelungsfeder 5 schräg an die Verriegelungsnut 4 eines liegenden Paneels 1 angesetzt ist, formschlüssig miteinander verriegeln lassen. Hierfür wird üblicherweise ein Paneel schräg angesetzt, wie durch die als Strich-Punkt-Linie angedeutete Paneelkante in Fig. 1a verdeutlicht, wobei das gleiche für die folgenden Figurengruppen 2 bis 15 gilt, in denen ebenfalls eine schräg angesetzte Paneelkante als Strich-Punkt-Linie angedeutet ist. Durch diese Art der Verriegelung mittels einer Schwenkbewegung M ist jeweils die dargestellte geschlossene Stoßfuge T zweier Paneelkanten 1 und 1' erzeugbar. Durch die beiden Stoßflächen 8 und 9, die miteinander in Kontakt gebracht sind, ist ein erstes Bereichsende eines Bewegungsspielraums X definiert, und zwar für eine Bewegung der Paneelkanten relativ zueinander und in einer Bewegungsrichtung, die sowohl senkrecht zu den formschlüssig verbundenen Paneelkanten als auch parallel zur Ebene der verbundenen Paneele liegt, wie durch den Doppelpfeil C angedeutet.

Außerdem weist jedes der Halteprofile 3 beziehungsweise 3' je eine untere Anschlagfläche auf. Eine untere Anschlagfläche 10 des Paneels 1, das mit der Verriegelungsnut 4 versehen ist, befindet sich an der unteren Nutwand 4b der Verriegelungsnut 4. Zu diesem Zweck ist die untere Nutwand 4b an ihrem freien Ende mit einem Halterand 11 versehen, der zur Paneeloberfläche S' gerichtet ist. Der Halterand 11 weist eine zum Nutgrund der Verriegelungsnut 4 gerichtete freie Seite auf, an welcher die untere Anschlagfläche 10 ausgebildet ist.

In dem Ausführungsbeispiel der Figuren 1a/1b ist diese untere Anschlagfläche 10 im Verhältnis zum Lot auf der Paneeloberfläche mit einer Neigung um einen Winkel α angeordnet. Eine untere Anschlagfläche 12 des Paneels 1', das mit der Verriegelungsfeder 5 versehen ist, befindet sich an der Federunterseite 13. Diese weist eine Hinterschneidung 14 auf und bildet so eine rückwärtige zum Kern des Paneels gerichtete Seite, an der die untere Anschlagfläche 12 ausgebildet ist. Diese untere Anschlagfläche 12 der Verriegelungsfeder 5 ist um den gleichen Winkel α im Verhältnis zum Lot auf der Paneeloberfläche S/S' geneigt wie die untere Anschlagfläche 10 der Verriegelungsnut 4.

Die geneigte Position der unteren Anschlagfläche 10 der Verriegelungsnut 4 bildet eine schiefe Ebene und die untere Nutwand 4b weist eine gewisse Federelastizität auf. Wenn die untere Nutwand 4b elastisch nach unten gebogen ist, dann entsteht in der unteren Nutwand 4b eine federelastische Rückstellkraft. Wenn diese Rückstellkraft gegen die untere Anschlagfläche 12 der Verriegelungsfeder 5 drückt, können dadurch die Paneelkanten 1 und 1' aufeinander zu bewegt werden, so dass sich ein vorhandener Spalt B zwischen den oberen Stoßflächen 8 und 9 verengt. Vorzugsweise ist der alleine durch die Rückstellkraft erzielbare Weg der Paneelkanten aufeinander zu nicht so groß, als dass die oberen Stoßflächen 8 und 9 mit einander in Kontakt kommen könnten.

Wenn die oberen Stoßflächen 8 und 9 einander berühren und die Stoßfuge T geschlossen ist, dann sind die unteren Anschlagflächen 10 und 12 maximal voneinander beabstandet. Der in Fig. 1a dargestellte horizontale Abstand A zwischen den unteren Anschlagflächen 10 und 12 bemisst die Größe des Bewegungsspielraums X für die Paneelkanten 1 beziehungsweise 1' im verriegelten Zustand (A = X). Wenn der Abstand A zwischen den unteren Anschlagflächen 10 und 12 als Hypotenuse eines rechtwinkligen Dreiecks aufgefasst wird, dann entspricht das Maß des Spaltes B (Spaltweite) einer der Katheten dieses rechtwinkligen Dreiecks.

In Fig. 1b sind die beiden Paneelkanten 1 und 1' relativ zueinander verschoben. Die unteren Anschlagflächen 10 und 12 berühren einander und zwischen den oberen Stoßflächen 8 und 9 hat sich ein Spalt A gebildet. Die unteren Anschlagflächen 10 und 12 definieren in dieser Position, in der sie einander berühren, das zweite Bereichsende des Bewegungsspielraums X für die verriegelten Paneelkanten 1/1'.

An einer Federoberseite 15 der Verriegelungsfeder 5 ist ein Abschnitt 15a vorgesehen, der sich parallel zur Paneeloberfläche S' erstreckt (horizontal). Dieser Abschnitt 15a bewirkt zusammen mit der Verriegelungsnut 4 im Wesentlichen die Festigkeit der Verriegelung in vertikaler Richtung und hält die Paneeloberflächen S und S' außerdem in einer Ebene und verhindert einen unerwünschten Höhenversatz zwischen den Paneeloberflächen S und S'.

Oberhalb dieses Abschnitts 15a ist die obere Stoßfläche 9 angeordnet und oberhalb der Stoßfläche 9 befindet sich die Kantenbrechung K₂ in Form einer Fase 16. Die Neigung der Fase 16 und die Neigung der oberen Stoßfläche 9 sind gleich. Die Kantenbrechung K₁ ist ebenfalls als Fase 17 ausgebildet. Die beiden Fasen 16 und 17 bilden eine symmetrische V-Fuge 18. Beide Paneeloberflächen S und S' liegen bei diesem Ausführungsbeispiel in einer gemeinsamen Ebene. Es besteht also kein Höhenversatz an den Paneelkanten beziehungsweise zwischen den Paneeloberflächen S/S'. Selbstverständlich kann anstelle von Fasen 16 und 17 auch eine andere Gestaltung der Kantenbrechung vorgesehen sein, wie ein Radius oder eine Stufe und selbstverständlich können Kantenbrechungen, die eine gemeinsame Fuge bilden, auch unsymmetrisch zueinander angeordnet sein oder unterschiedliche geometrische Formen aufweisen, beispielsweise kann eine Kantenbrechung in Form einer Fase mit einer Kantenbrechung in Form eines Radius kombiniert sein.

Die Federunterseite 13 weist eine ebene untere Kontaktfläche 13a auf, die parallel zur Paneeloberfläche S' angeordnet ist. Die untere Nutwand 4b der Verriegelungsnut 4 hat eine Innenseite 19, die mit einer ebenfalls zur Paneeloberfläche S parallel angeordneten ebenen Auflagefläche 19a für die untere Kontaktfläche 13a der Verriegelungsfeder 5 versehen ist. Von oben auf die Paneeloberfläche S/S' einwirkende Kräfte können durch die ebene Kontaktfläche 13a der Verriegelungsfeder 5 in die Auflagefläche 19a der unteren Nutwand 4b abgeleitet werden. Darüber hinaus dienen die Kontaktfläche 13a und die Auflagefläche 19a zur Lagerung und Führung für die Paneelkanten, welche im Rahmen des vorhandenen Bewegungsspielraums X in Bewegung sein können und ihre relative Position zueinander verändern können.

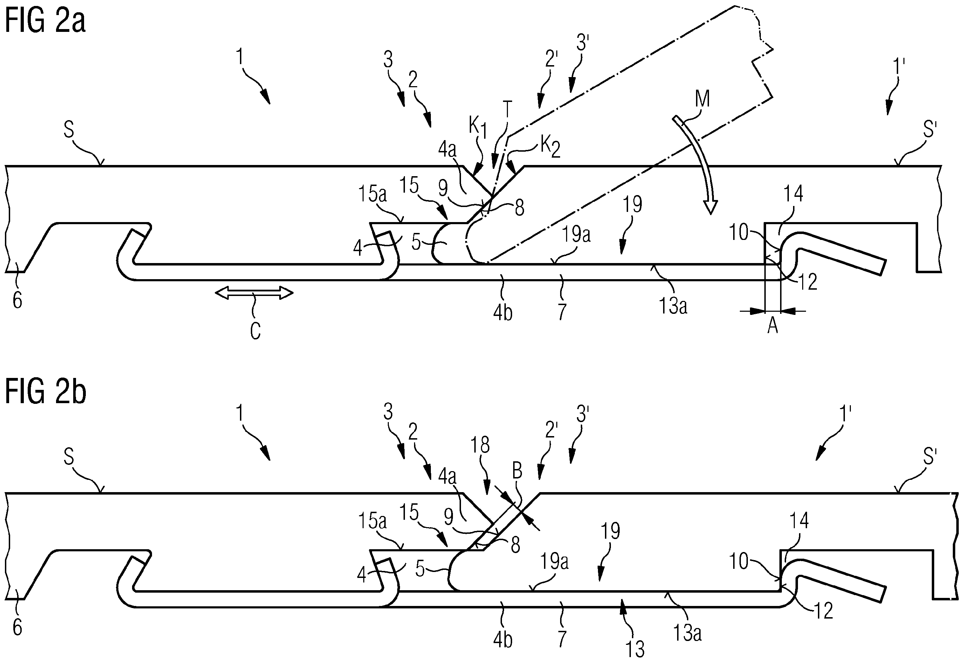

Eine Alternative, die auf dem Ausführungsbeispiel der Figuren 1a/1b basiert ist in den Figuren 2a und 2b gezeigt. Auf die Figuren 1a/1b wird Bezug genommen. Für gleiche Merkmale werden die gleichen Bezugszeichen verwendet. Die Alternative unterscheidet sich vom vorherigen Ausführungsbeispiel dadurch, dass die beiden unteren Anschlagflächen 10 beziehungsweise 12 anders angeordnet sind. Die untere Anschlagfläche 10, die sich am Halterand 11 der unteren Nutwand 4b der Verriegelungsnut 4 befindet, erstreckt sich parallel zum Lot auf der Paneeloberfläche. Auf eine Neigung gegenüber dem Lot wird verzichtet, um bei dieser Ausführung den Effekt einer schiefen Ebene zu vermeiden. Die untere Nutwand der Verriegelungsnut 4 ist auch bei diesem Ausführungsbeispiel federelastisch und kann nach unten gebogen werden. Die federelastische Rückstellkraft kann die untere Nutwand 4b dann wieder in Richtung ihrer neutralen Position zurückstellen. Da die untere Anschlagfläche 10 aber keine schiefe Ebene bildet, wird diese Rückstellbewegung nicht in eine horizontale Bewegung der Paneelkante 1' mit der Verriegelungsfeder 5 umgelenkt und die Paneelkanten 1 und 1' werden nicht aufeinander zu bewegt.

Ein drittes Ausführungsbeispiel ist in den Figuren 3a und 3b gezeigt. Dieses Ausführungsbeispiel hat mit den vorherigen Ausführungsbeispielen die Gestaltung der Kantenbrechungen K₁ und K₂ gemein und an der Federoberseite 15 der Verriegelungsfeder 5 ist wiederum ein Abschnitt 15a, der sich in der dargestellten Gebrauchslage horizontal erstreckt, das heißt parallel zur Paneeloberfläche S'. Dieser Abschnitt 15a bewirkt zusammen mit der Verriegelungsnut 4 im Wesentlichen die Festigkeit der Verriegelung in vertikaler Richtung und hält die Paneeloberflächen S und S' außerdem in einer gemeinsamen Ebene und verhindert einen unerwünschten Höhenversatz zwischen den Paneeloberflächen S und S'. An ihrer Federunterseite 13 ist die Verriegelungsfeder 5 mit einer Kontaktfläche 13a versehen, die mit einer Krümmung nach außen gewölbt ist, während die unter Nutwand 4b der Verriegelungsnut 4 an ihrer Innenseite 19 eine Auflagefläche 19a und an ihrem freien Ende einen Halterand 11 hat, wobei die Auflagefläche 19a eine nach innen gerichtete Krümmung aufweist, die zum Halterand 11 hin ansteigt und dort die untere Anschlagfläche 10 bildet. Die Kontur der Krümmung der Auflagefläche 19a weist eine weitere (offenere) Gestalt auf als die Krümmungskontur der Kontaktfläche 13a der Federunterseite 13, so dass sich dadurch ein Bewegungsspielraum X für die Paneelkanten 1 und 1' ergibt. Von oben auf das Paneel 1 beziehungsweise 1' einwirkende Kräfte können durch die gekrümmte Kontaktfläche 13a der Verriegelungsfeder 5 in die gekrümmte Auflagefläche 19a der unteren Nutwand 4b abgeleitet werden. Die unteren Anschlagflächen 10 und 12 sind jeweils Teil der Krümmung der Federunterseite 13a respektive der innenseitigen Krümmung der unteren Nutwand 4b. In Fig. 3b sind die Paneelkanten 1 und 1' auseinander bewegt. Die unteren Anschlagflächen 10 und 12 berühren einander und zwischen den oberen Stoßflächen 8 und 9 hat sich ein Spalt B gebildet. Bei diesem Ausführungsbeispiel kann, begünstigt durch ihre Krümmung, die untere Nutwand 4b elastisch nach unten gebogen werden und eine Rückstellkraft entstehen, welche die Paneelkanten 1 und 1' wieder aufeinander zu bewegt. Wenn die untere Nutwand 4b wieder ihre neutrale Position erreicht hat, bleibt bei diesem Ausführungsbeispiel dennoch ein Restspalt zwischen oberen Stoßflächen 8 und 9 bestehen.

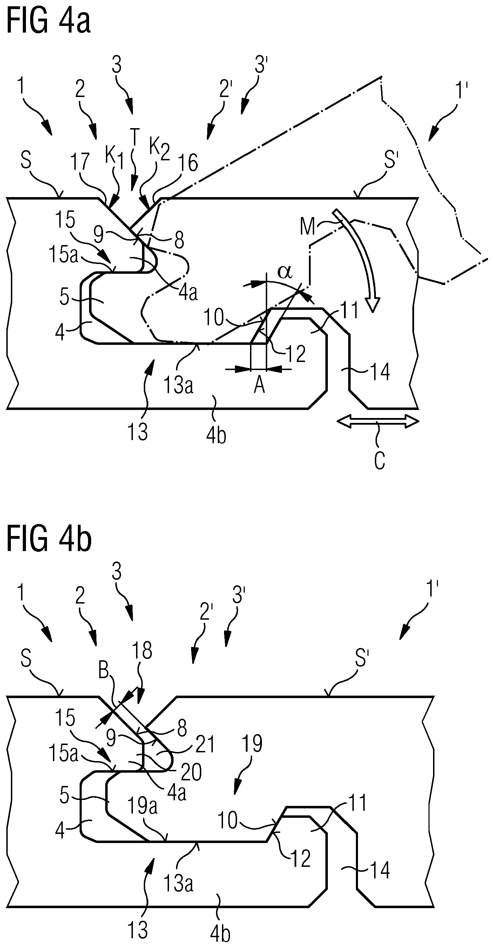

Die Figuren 4a und 4b zeigen ein weiteres Ausführungsbeispiel mit einer Verriegelungsfeder 5 und einer komplementären Verriegelungsnut 4, wobei die Verriegelungsfeder 5 an ihrer Federoberseite 15 einen Abschnitt 15a hat, der sich in der dargestellten Gebrauchslage horizontal, beziehungswiese parallel zur Paneeloberfläche S/S' erstreckt. Zusammen mit der komplementären Verriegelungsnut 4 bewirkt dieser Abschnitt der Verriegelungsfeder 5 im Wesentlichen die Festigkeit der Verriegelung in vertikaler Richtung. Er hält außerdem die Paneeloberflächen S und S' in einer gemeinsamen Ebene und verhindert einen unerwünschten Höhenversatz zwischen den Paneeloberflächen. Es sind obere Stoßflächen 8 und 9 vorgesehen, die jedoch an anderer Stelle angeordnet sind, als bei den vorherigen Ausführungsbeispielen. Die obere Stoßfläche 8 derjenigen Paneelkante 1, welche die Verriegelungsnut 4 hat, befindet sich an der Außenseite des freien Endes der oberen Nutwand 4a der Verriegelungsnut 4, und sie liegt in einer Flucht beziehungswiese in einer Ebene mit der Fase 17. Das freie Ende der oberen Nutwand 4a wirkt wie eine Feder und passt in eine dafür vorgesehen Nut, die an dem komplementären Halteprofil 3' oberhalb von dessen Verriegelungsfeder 5 angeordnet ist. Die zusätzliche Feder wird im Sinne der Erfindung als Verschlussfeder 20 bezeichnet und die zusätzliche Nut als Verschlussnut 21, weil mittels ihrer Stoßflächen 8 und 9 eine geschlossene Stoßfuge T erzeugbar ist. Bei dieser Gestaltung hat demzufolge jedes der Halteprofile 3 beziehungsweise 3' je eine Nut und je eine Feder, das heißt, im verriegelten Zustand sind zwei Nuten, nämlich Verriegelungsnut 4 und Verschlussnut 21, sowie zwei Federn, nämlich Verriegelungsfeder 5 und Verschlussfeder 20, an der Verriegelung der Paneelkanten 1 und 1' beteiligt.

Das Ausführungsbeispiel der Figuren 4a und 4b sieht außerdem vor, dass die Federunterseite 13 der Verriegelungsfeder 5 eine ebene Kontaktfläche 13a hat, die parallel zur Paneeloberfläche S' angeordnet ist. Die untere Nutwand 4b der Verriegelungsnut 4 ist bei diesem Ausführungsbeispiel einstückig mit dem Kern des Paneels ausgebildet, und die untere Nutwand 4b weist ebenfalls eine zur Paneeloberfläche S parallel angeordnete ebene Auflagefläche 19a für die untere Kontaktfläche 13a der Verriegelungsfeder 5 auf. Von oben auf das Paneel 1/1' einwirkende Kräfte können durch die ebene Kontaktfläche 13a der Verriegelungsfeder 5 in die Auflagefläche 19a der unteren Nutwand 4b abgeleitet werden. Darüber hinaus dienen die Kontaktfläche 13a und die Auflagefläche 19a zur Lagerung und Führung für die Paneelkanten 1 und 1', welche im Rahmen eines vorhandenen Bewegungsspielraums X in Bewegung sein und ihre relative Position zueinander verändern können. Den Bewegungsspielraum X begrenzen einerseits die oberen Stoßflächen 8 und 9, die sich an der Verschlussfeder 20 beziehungsweise der Verschlussnut 21 befinden, während das andere Bereichsende durch die unteren Anlageflächen 10 und 12 begrenzt wird, welche sich an der Verriegelungsfeder 5 beziehungsweise an der Verriegelungsnut 4 befinden. Die Kantenbrechungen K₁ und K₂ sind wiederum als Fasen 16 beziehungsweise 17 gestaltet und bilden gemeinsam eine symmetrische V-Fuge 18.

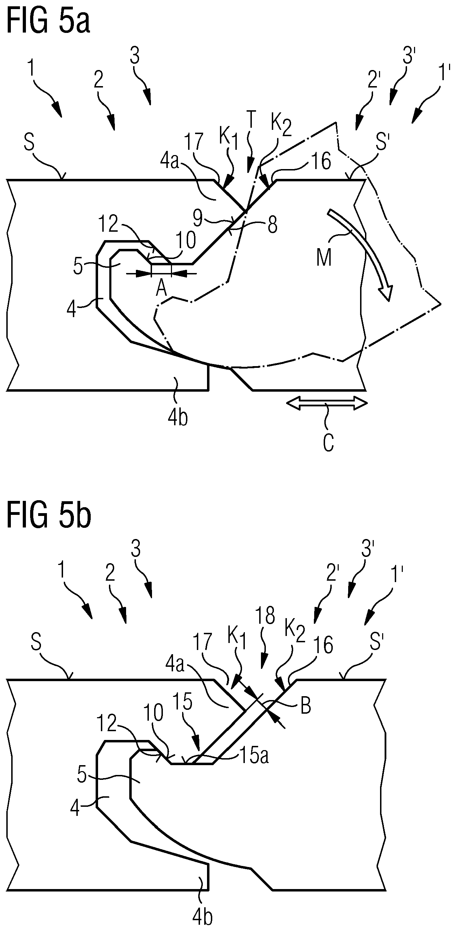

Fig. 5a und 5b zeigen ein Ausführungsbeispiel, bei dem die unteren Anlageflächen 10 und 12, welche einem Auseinanderbewegen der Paneelkanten 1 und 1' entgegenwirken, an einer andere Stelle angeordnet sind als bei den vorherigen Ausführungsbeispielen, und zwar an einer Innenseite 22 der oberen Nutwand 4a der Verriegelungsnut 4 beziehungsweise an der Federoberseite 15 der Verriegelungsfeder 5. Die untere Nutwand 4b ist kürzer ausgelegt als die obere Nutwand 4a der Verriegelungsnut 4.

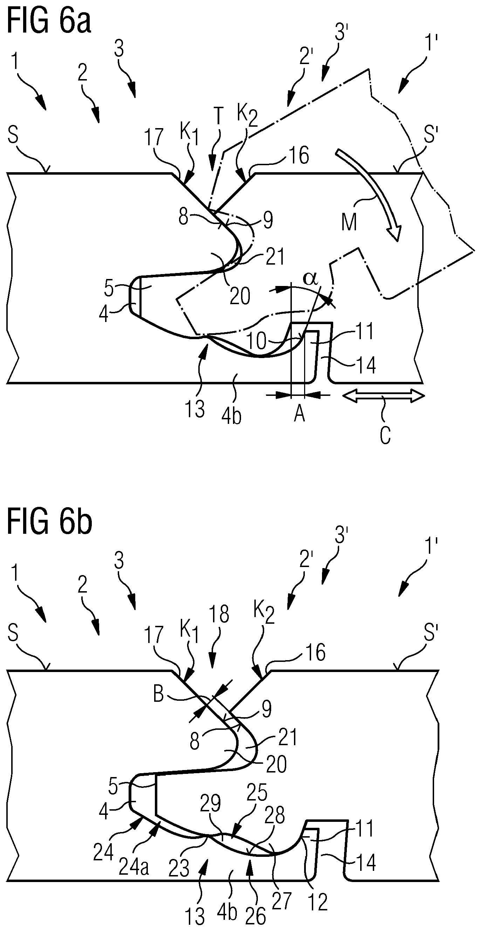

Ein alternatives Ausführungsbeispiel zeigen die Figuren 6a und 6b. Es handelt sich um ein weiteres Ausführungsbeispiel, bei dem jedes der Halteprofile 3 und 3' je eine Nut und je eine Feder aufweist wie beim Ausführungsbeispiel gemäß den Figuren 4a/4b, auf die Bezug genommen wird. Das heißt, eines der Halteprofile 3 hat eine Verriegelungsnut 4 und gleichzeitig eine Verschlussfeder 20, und das komplementäre Halteprofil 3' weist eine Verriegelungsfeder 5 und gleichzeitig eine Verschlussnut 21 auf.

Die Federunterseite 13 der Verriegelungsfeder 5 weist eine Kontur mit einem Knick 23 auf. Ein vor dem Knick 23 liegender und zur Spitze der Verriegelungsfeder 5 weisender vorderer Bereich 24 der Federunterseite 13 hat eine gekrümmte Kontaktfläche 24a, die zur Federspitze hin ansteigt. Dazu passend hat die Verriegelungsnut 4 eine Auflagefläche 25 mit zwei Bereichen, wobei ein Bereich 25a dem Nutgrund der Verriegelungsnut 4 zugewandt ist und zum Nutgrund hin aufsteigt. Dieser Aufstiegsbereich 25a der Auflagefläche 25 wirkt im verriegelten Zustand mit der gekrümmten Kontaktfläche 24a des vorderen Bereichs 24 der Federunterseite 13 zusammen. Ein hinterer Bereich 26 der Federunterseite 13 bildet einen nach außen gewölbten Vorsprung 27, der in eine Vertiefung 28 in der Auflagefläche 25 hineinragt und auf der Auflagefläche 25 lagert. Zwischen der vorderen gekrümmten Kontaktfläche 24a der Federunterseite 13 und jener Stelle, an welcher der gewölbte Vorsprung 27 die Vertiefung 28 berührt, befindet sich ein Hohlraum 29, in dem Abrieb- und oder Schmutzpartikel Platz finden können.

An einer rückwärtigen Fläche des Vorsprungs 27 befindet sich die untere Anlagefläche 12 der Verriegelungsfeder 5. Dazu passend ist an dem freien Ende der unteren Nutwand 4b ein Halterand 11 vorgesehen, der zur Paneeloberfläche S' gerichtet ist. Der Halterand 11 weist eine zum Nutgrund der Verriegelungsnut 4 gerichtete freie Seite auf, an welcher sich die untere Anschlagfläche 10 der Verriegelungsnut 4 befindet, die mit der unteren Anlagefläche 12 der Verriegelungsfeder zusammenwirkt. Die untere Anschlagfläche 10 der Verriegelungsnut 4 ist im Verhältnis zum Lot auf der Paneeloberfläche mit einer Neigung um einen Winkel α angeordnet. In Fig. 6a haben die unteren Anlageflächen 10 und 12 einen horizontalen Abstand A zueinander und die oberen Stoßflächen 8 und 9 berühren einander wie im Beispiel gemäß Fig. 4a.

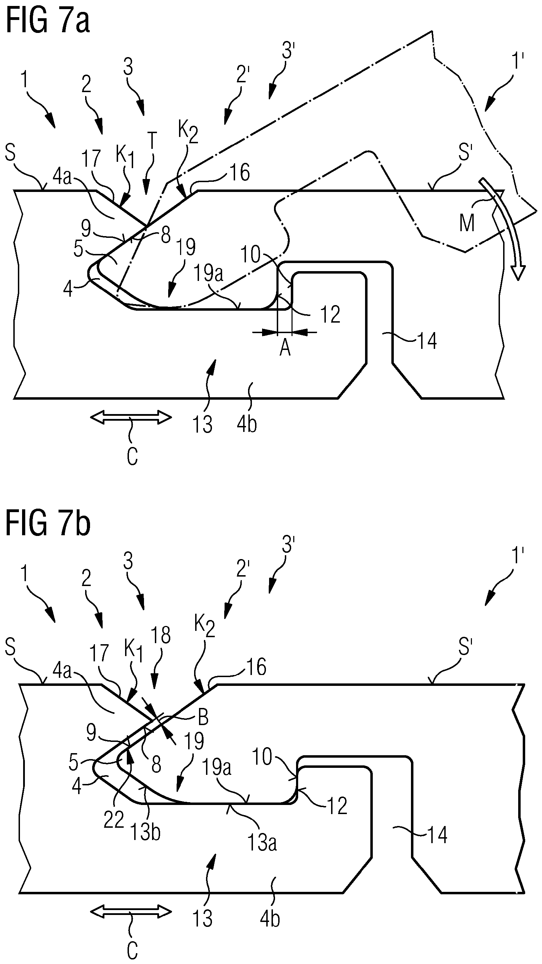

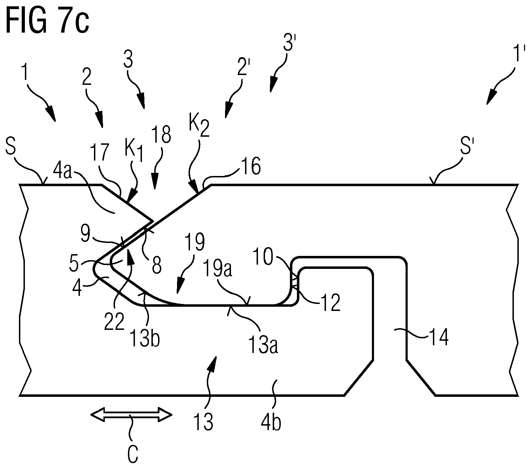

Die Figuren 7a, 7b und 7c zeigen ein Paneel mit einer besonders einfachen Konstruktion der Paneelkanten 1 und 1'. Es weist eine Verriegelungsfeder 5 und eine Verriegelungsnut 4 auf sowie Kantenbrechungen K₁ und K₂ an beiden einander gegenüberliegenden Paneelkanten. Es sind obere Stoßflächen 8 und 9 sowie untere Anschlagflächen 10 und 12 vorgesehen. Im verriegelten Zustand ist ein Bewegungsspielraum X vorhanden, damit eine Bewegung der Paneelkanten relativ zueinander möglich ist und zwar in einer Bewegungsrichtung, die sowohl senkrecht zu den formschlüssig verbundenen Paneelkanten als auch parallel zur Ebene der verbundenen Paneele liegt. Die Kantenbrechungen K₁ und K₂ sind jeweils als Fase 16 beziehungsweise 17 gestaltet. An der Fase 16 jener Paneelkante 1', welche die Verriegelungsfeder 5 aufweist, schließt sich die obere Stoßfläche 9 an. Diese Stoßfläche 9 liegt in einer gemeinsamen Ebene mit der Fase 16. Dies führt bei diesem Ausführungsbeispiel zu der Besonderheit, dass die obere Stoßfläche 9 integraler Bestandteil der Federoberseite 15 der Verriegelungsfeder 5 ist. Außerdem hat die Verriegelungsnut 4 eine obere Nutwand 4a, deren Innenseite 22 an den Neigungswinkel der Federoberseite 15 angepasst ist. Die Innenseite 22 der oberen Nutwand 4a bildet somit die obere Stoßfläche 8 als integralen Bestandteil der Innenseite 22 der Verriegelungsnut 4. Die Festigkeit der Verriegelung in vertikaler Richtung wird im Wesentlichen durch die geneigte Federoberseite 15 zusammen mit der ebenfalls geneigten oberen Innenseite 22 der Verriegelungsnut 4 bewirkt. Außerdem ist wegen der integralen Bauweise vermittels der geneigten Federoberseite 15 und der Innenseite 22 der Verriegelungsnut 4 gleichzeitig auch eine geschlossene Stoßfuge T erzeugbar. Darüber hinaus wird gewährleistet, dass die Paneeloberflächen S und S' in einer Ebene liegen und es wird auf diese Weise einem unerwünschten Höhenversatz zwischen den Paneeloberflächen S und S' entgegengewirkt.

Die untere Anschlagfläche 10 des Paneels, das mit der Verriegelungsnut 4 versehen ist, befindet sich an der unteren Nutwand 4b der Verriegelungsnut 4. Zu diesem Zweck ist die untere Nutwand 4b an ihrem freien Ende mit einem Halterand 11 versehen, der aufwärts zur Paneeloberfläche S gerichtet ist. Der Halterand 11 weist eine zum Nutgrund der Verriegelungsnut 4 gerichtete freie Seite auf, an welcher die untere Anschlagfläche 10 sich in einer Richtung senkrecht zur Paneelebene S erstreckt. Die untere Anschlagfläche 12 des Paneels, das mit der Verriegelungsfeder 5 versehen ist, befindet sich an der Federunterseite 13. Diese weist eine Hinterschneidung 14 auf und bildet so eine rückwärtige zum Kern des Paneels gerichtete Seite, an der die untere Anschlagfläche 12 ausgebildet ist. Diese untere Anschlagfläche 12 der Verriegelungsfeder 5 erstreckt sich senkrecht zur Paneeloberfläche S'.

In Fig. 7a berühren sich die oberen Stoßflächen 8 und 9 und bilden eine geschlossene Stoßfuge T. Die unteren Anschlagflächen 10 und 12 haben in der dargestellten Position der Paneelkanten einen horizontalen Abstand A voneinander, welcher dem Bewegungsspielraum X entspricht.

In Fig. 7c ist eine Position der Panelkanten 1 und 1' dargestellt, bei der sich weder die oberen Stoßflächen 8 und 9 berühren, noch die unteren Anlageflächen 10 und 12 miteinander in Kontakt sind. Die Paneelkanten sind miteinander verriegelt und können sich innerhalb des Bewegungsspielraums X relativ zueinander so bewegen, bis entweder das Bereichsende des Bewegungsspielraums erreicht ist, welches durch den Kontakt der oberen Stoßflächen 8 und 9 definiert ist, oder sie können sich jeweils in die entgegengesetzte Richtung bewegen, bis dasjenige Bereichsende des Bewegungsspielraums erreicht ist, welches durch die unteren Anschlagflächen 10 und 12 definiert ist. Im verlegten Zustand, wenn eine Vielzahl Paneele zu einem Fußbodenbelag zusammengefügt ist, wird sich zwischen zwei verriegelten Paneelkanten häufig eine relative Zwischenposition einstellen, wie in Fig. 7c angedeutet, bei der ein mehr oder weniger großer oberer Spalt vorliegt sowie eine mehr oder weniger große Lücke zwischen den unteren Anschlagflächen 10 und 12 der Paneelkanten entsteht. Während der alltäglichen Benutzung können sich die Paneelkanten relativ zueinander bewegen und die Größe des oberen Spaltes und die Lücke zwischen den unteren Anschlagflächen sich verändern. Eine Zwischenposition der Paneelkanten relativ zueinander, wie anhand der Figuren 7c, 8c und 10c exemplarisch dargestellt, ist selbstverständlich auch bei alle übrigen Ausführungsbeispielen möglich, wobei aber der Einfachheit halber auf zusätzliche Darstellungen für diese Ausführungsbeispiele verzichtet worden ist. Des Weiteren hat nach Fig. 7a die Innenseite 19 der unteren Nutwand 4b der Verriegelungsnut 4 eine horizontale Auflagefläche 19a, die sich parallel zur Paneeloberseite S erstreckt und einen vorderen Bereich 19b, der zum Nutgrund hin aufsteigt.

Die Verriegelungsfeder 5 hat an ihrer Federunterseite 13 eine horizontale Kontaktfläche 13a, die sich parallel zur Paneeloberseite S' erstreckt. Außerdem ist an der Federunterseite 13 ein ausgeprägter vorderer Bereich 13b vorgesehen, welcher zur Federspitze der Verriegelungsfeder 5 hin ansteigt. Im verriegelten Zustand findet zwischen dem aufsteigenden Bereich 19b der Innenseite 19 der unteren Nutwand sowie dem ansteigenden Bereich 13b der Federunterseite 13 keine Berührung statt. Lediglich die horizontalen Bereiche der Auflagefläche 19a und Kontaktfläche 13a liegen aufeinander.

Sofern bei den vorherigen und folgenden Ausführungsbeispielen eine Kantenbrechung an der Unterseite einer Paneelkante, respektive eines Paneels vorhanden ist, dient dies vorzugsweise zum Schutz der Kante vor Beschädigung.

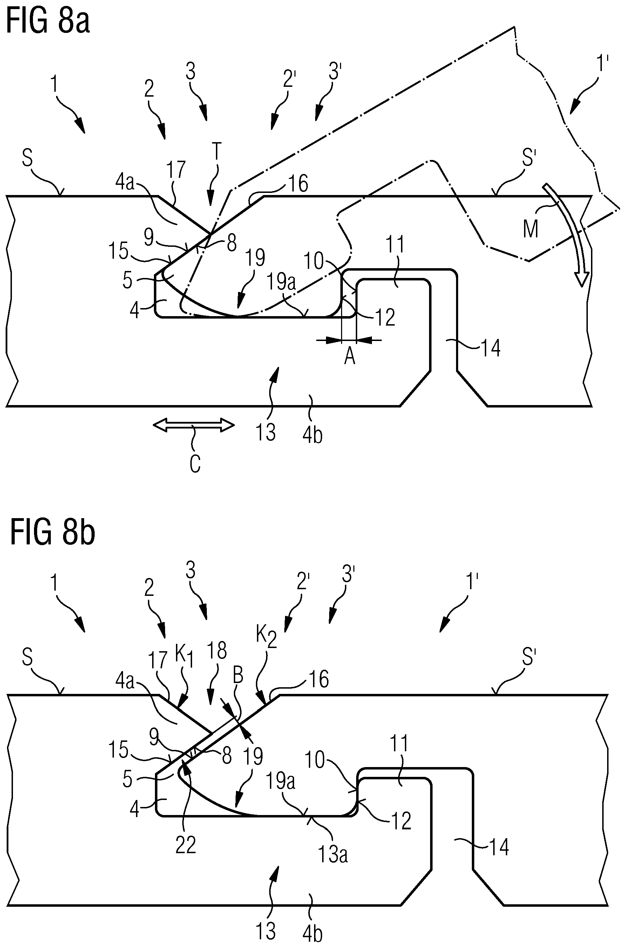

Das Ausführungsbeispiel der Figuren 8a und 8b basiert auf dem vorherigen Ausführungsbeispiel, auf das Bezug genommen wird. Im Vergleich zu diesem ist die Gestaltung der Verriegelungsnut 4 geändert. Das vorherige Ausführungsbeispiel hat einen zum Nutgrund der Verriegelungsnut 4 reichenden aufsteigenden Bereich 19b an der Innenseite 19 der unteren Nutwand 4b. Im Unterschied dazu wird beim vorliegenden Ausführungsbeispiel auf einen aufsteigenden Bereich an der Innenseite 19 verzichtet. Die Innenseite 19 erstreckt sich gemäß Fig. 8a im Wesentlichen bis zum Nutgrund der Verriegelungsnut parallel zur Paneeloberfläche (horizontal). Die Federunterseite 13 der Verriegelungsfeder 5 ist gleich geblieben, wie jene in Fig. 7a, das heißt, zur Federspitze der Verriegelungsfeder 5 hin ist ein ansteigender Bereich 13b an der Federunterseite 13 vorgesehen. Durch diese Änderung ist zwischen dem ansteigenden Bereich 13b der Federunterseite und der horizontalen Innenseite 19 mit der Auflagefläche 19a ein größerer Freiraum entstanden als beim Ausführungsbeispiel der Fig. 7a.

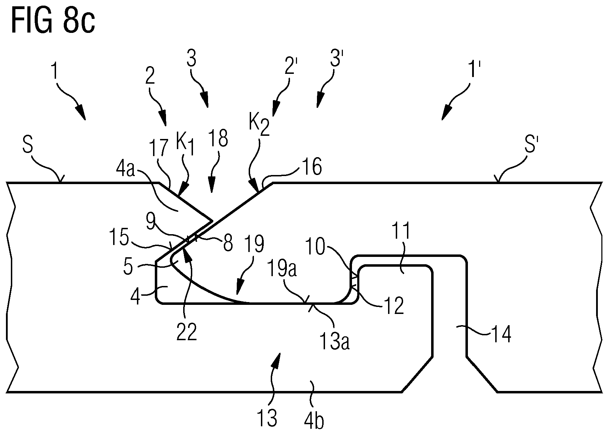

In Fig. 8c ist, wie in Fig. 7c eine Position der Panelkanten 1 und 1' dargestellt, bei der sich weder die oberen Stoßflächen 8 und 9 berühren, noch die unteren Anlageflächen 10 und 12 miteinander in Kontakt sind. Die Paneelkanten sind miteinander verriegelt und können sich innerhalb des Bewegungsspielraums X relativ zueinander so bewegen, bis entweder das Bereichsende des Bewegungsspielraums erreicht ist, welches durch den Kontakt der oberen Stoßflächen 8 und 9 definiert ist, oder sie können sich jeweils in die entgegengesetzte Richtung bewegen, bis dasjenige Bereichsende des Bewegungsspielraums erreicht ist, welches durch die unteren Anschlagflächen 10 und 12 definiert ist.

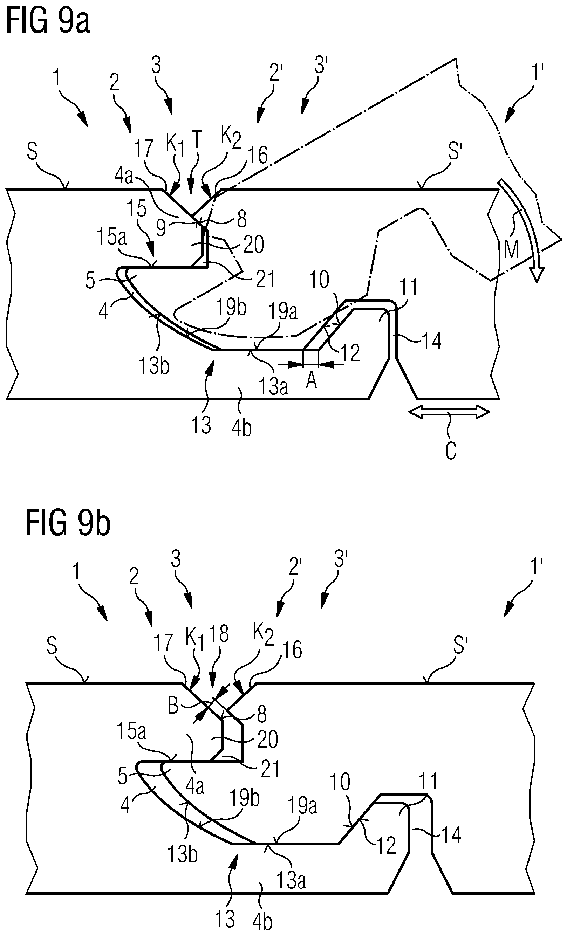

Die Figuren 9a und 9b zeigen ein drittes Ausführungsbeispiel eines Paneels, dessen komplementäre Halteprofile 3 und 3' je eine Nut und je eine Feder aufweisen, wie bei den Ausführungsbeispielen gemäß den Figuren 4a/4b sowie 6a/6b, auf die Bezug genommen wird. Das heißt, eines der Halteprofile 3 hat eine Verriegelungsnut 4 und gleichzeitig eine Verschlussfeder 20, während das komplementäre Halteprofil 3' eine Verriegelungsfeder 5 und gleichzeitig eine Verschlussnut 21 aufweist.

Das Paneel gemäß Fig. 9a unterscheidet sich vom Paneel gemäß Fig. 4a im Wesentlichen durch eine geänderte Gestaltung der Verriegelungsnut 4. Nach Fig. 9a weist die Verriegelungsnut 4 nämlich eine untere Nutwand 4b mit einer Innenseite 19 auf, die eine ausgeprägten aufsteigenden Bereich 19b aufweist. Der aufsteigende Bereich 19b steigt in Richtung des Nutgrundes der Verriegelungsnut 4 hin aufwärts. Außerdem hat die Innenseite 19 eine Auflagefläche 19a, die sich parallel zur Paneeloberfläche S erstreckt (horizontal). Der aufsteigende Bereich 19b endet etwa unterhalb des freien Endes der oberen Nutwand 4a der Verriegelungsnut 4. Die Federunterseite 13 hat eine Kontaktfläche 13a, die sich parallel zur Paneeloberfläche erstreckt sowie einen vorderen Bereich 13b, der zur Federspitze der Verriegelungsfeder 5 hin ansteigt.

Die unteren Anschlagflächen 10 und 12 sind, wie jene in Fig. 4a im Verhältnis zum Lot auf der Paneeloberfläche S/S' geneigt angeordnet.

Es sind obere Stoßflächen vorgesehen und zwar ist die obere Stoßfläche 8 derjenigen Paneelkante 1, welche die Verriegelungsnut 4 hat, an der Außenseite des freien Endes der oberen Nutwand 4a der Verriegelungsnut 4 vorgesehen. Dieses freie Ende der oberen Nutwand 4a, wirkt wie eine Feder und passt in eine dafür vorgesehen Nut, die an dem komplementären Halteprofil 3' oberhalb von dessen Verriegelungsfeder 5 angeordnet ist. In dieser Nut ist an einer oberen Nutwand die komplementäre Stoßfläche 9 angeordnet. Die zusätzliche Feder wird im Sinne der Erfindung wiederum als Verschlussfeder 20 bezeichnet und die zusätzliche Nut als Verschlussnut 21, weil sie die Stoßflächen 8 und 9 tragen, welche idealerweise die Stoßfuge T schließen.

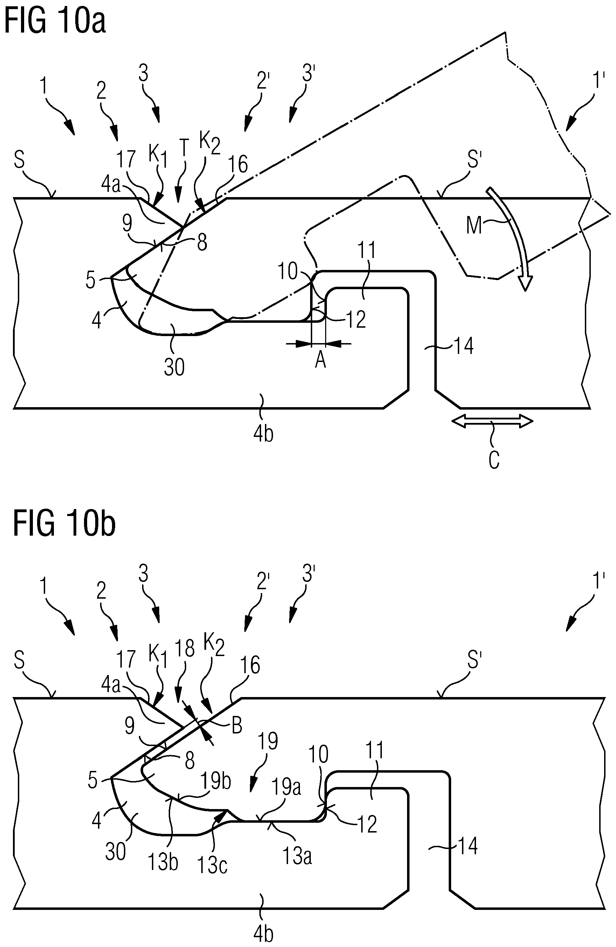

Die Figuren 10a und 10b stellen ein Ausführungsbeispiel dar, das auf dem Ausführungsbeispiel der Figuren 7a/7b basiert, auf das Bezug genommen wird. Im Unterschied zu diesem ist im Wesentlichen die Innenseite 19 der unteren Nutwand 4b der Verriegelungsnut 4 geändert. Die Innenseite 19 der unteren Nutwand 4b hat im Wesentlichen zwei Bereiche. Einen ersten Bereich bildet eine Auflagefläche 19a, welche sich parallel zur Paneeloberseite S erstreckt. Diese Auflagefläche 19a reicht bis an eine untere Anschlagfläche 10 heran, welche an einem Halterand 11 der unteren Nutwand 4b vorgesehen ist. Die Auflagefläche 19a erstreckt sich von dort in Richtung des Nutgrundes der Verriegelungsnut 4, wobei sie jedoch nicht bis an den Nutgrund heranreicht. Zwischen dem Ende Auflagefläche 19a und dem Nutgrund ist eine Aussparung 19c vorgesehen, die tiefer liegt als die Auflagefläche 19a. Auch die Federunterseite 13 der Verriegelungsfeder 5 weist im Wesentlichen zwei Bereiche auf. Einen ersten Bereich bildet eine Kontaktfläche 13a, die sich parallel zur Paneeloberseite erstreckt, und die mit der Auflagefläche 19a der unteren Nutwand 46 der Verriegelungsnut 4 zusammenwirkt. Ein zweiter Bereich 13b der Federunterseite 13 ist der Spitze der Verriegelungsfeder 5 zugewandt. Dieser zweite Bereich hat einen Absatz 13c, welcher an der Kontaktfläche 13a ansetzt und an den sich ein gekrümmter ansteigender Bereich 13b anschließt, der zur Spitze der Verriegelungsfeder 5 hin ansteigt. Der Absatz 13c steht gegenüber der Kontaktfläche 13a zurück, das heißt, sein Abstand zur Paneeloberfläche S' ist geringer als jener der Kontaktfläche 13a. Durch den Absatz 13c an der Federunterseite 13 sowie durch die Aussparung 19c in der unteren Nutwand 4b ist im verriegelten Zustand ein vergrößerter Freiraum 30 geschaffen. Durch den Freiraum 30 ist es möglich, dasjenige Paneel 1' mit der Verriegelungsfeder 5 schräg anzusetzen, wie mittels der strichpunktierten Linie in Fig. 10a dargestellt, ohne die Halteprofile 3 und/oder 3' elastisch verformen zu müssen. Die Spitze der Verriegelungsfeder 5, respektive deren ansteigender vorderer Bereich 13b kann in dieser Schrägstellung des Paneels 1' abgesenkt werden bis auf den Boden der Aussparung 19c der unteren Nutwand 4b, wie durch die als Strich-Punkt-Linie angedeutete schräggestellte Paneelkante verdeutlicht.

Die unteren Anschlagflächen 10 und 12 erstrecken sich gemäß den Figuren 10a und 10b ebenso in einer Richtung senkrecht zur Paneelebene, wie in Fig. 7a/7b.

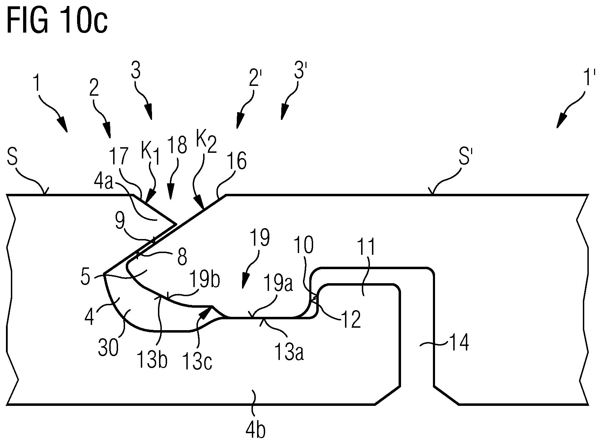

In Fig. 10c ist eine Position der Panelkanten 1 und 1' dargestellt, bei der sich weder die oberen Stoßflächen 8 und 9 berühren, noch die unteren Anlageflächen 10 und 12 miteinander in Kontakt sind. Die Paneelkanten sind miteinander verriegelt und können sich innerhalb des Bewegungsspielraums X relativ zueinander so bewegen, bis entweder das Bereichsende des Bewegungsspielraums erreicht ist, welches durch den Kontakt der oberen Stoßflächen 8 und 9 definiert ist, oder sie können sich jeweils in die entgegengesetzte Richtung bewegen, bis dasjenige Bereichsende des Bewegungsspielraums erreicht ist, welches durch die unteren Anschlagflächen 10 und 12 definiert ist. Die

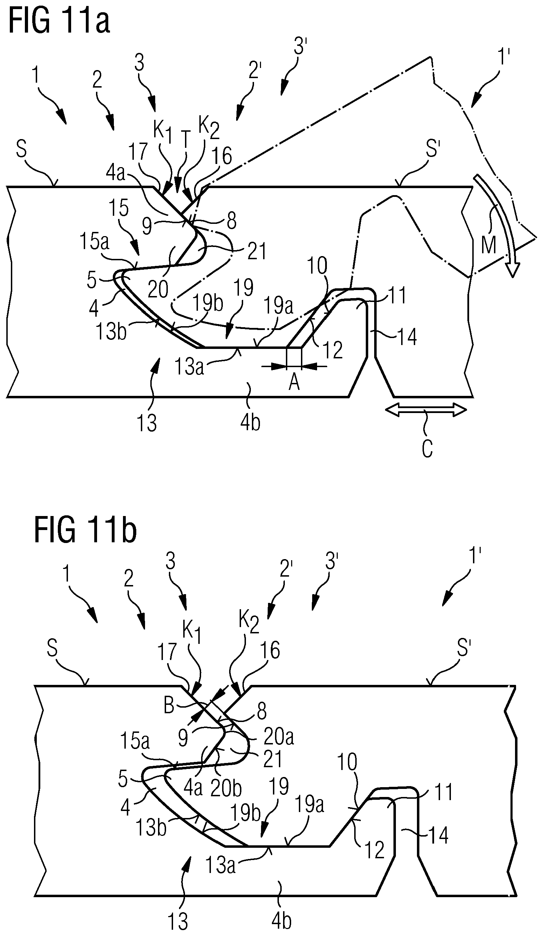

Die Figuren 11a und 11b zeigen ein Ausführungsbeispiel, das auf dem Ausführungsbeispiel der Figuren 9a/9b basiert, auf das Bezug genommen wird. Wie bei diesem weisen die komplementären Halteprofile 3und 3' je eine Nut und je eine Feder auf. Das heißt, eines der Halteprofile 3 hat eine Verriegelungsnut 4 und gleichzeitig eine Verschlussfeder 20 während das komplementäre Halteprofil 3' eine Verriegelungsfeder 5 und gleichzeitig eine Verschlussnut 21 aufweist.

Gegenüber dem Ausführungsbeispiel der Figurengruppe 9 ist die Federoberseite 15 der Verriegelungsfeder 5 nicht mehr parallel zur Paneeloberfläche S' angeordnet, sondern sie ist gemäß Fig. 11a gegenüber der Paneeloberfläche S' in einem flachen Winkel geneigt. Das gleiche gilt für die Innenseite 22 der oberen Nutwand 4a der Verriegelungsnut 4, deren Neigungswinkel im verriegelten Zustand mit dem Neigungswinkel der Federoberseite 15 korrespondiert.

Die obere Nutwand 4a der Verriegelungsnut 4 bildet außerdem die Verschlussfeder 20, welche bei diesem Ausführungsbeispiel eine Federspitze 20a und zwei Seitenflächen hat, von denen die obere Seitenfläche gleichzeitig die obere Stoßfläche 8 bildet. Die untere Seitenfläche 20b ist gegenüber der Paneeloberfläche S stärker geneigt als der übrige Teil der Innenseite 22 der oberen Nutwand 4a, dessen Neigungswinkel mit jenem der Federoberseite 15 korrespondiert. Zwischen der unteren Seitenfläche 20b und dem Nutgrund bildet sich ein Freiraum, wenn zwei Paneelkanten 1 und 1' zusammengefügt sind, wobei der Nutgrund hier U-förmig rund gestaltet ist.

Wenn, wie in Fig. 11b dargestellt, zwei verriegelte Paneelkanten 1 und 1' eine Position einnehmen, in der sich zwischen den oberen Stoßflächen 8 und 9 ein Spalt ergibt, dann entsteht auch ein minimaler Spalt zwischen der flach geneigten Federoberseite 15 und der Innenseite 22 der oberen Nutwand 4a der Verriegelungsnut 4.

Die Figurengruppen 12 bis 15 zeigen vier unterschiedliche Ausführungsbeispiele, die ein identisches Grundkonzept haben und sich lediglich durch die Gestaltung im Bereich der Kantenbrechung K₁ beziehungsweise K₂ an der Paneeloberfläche S beziehungsweise S' sowie durch die Gestaltung der oberen Stoßflächen 8 und 9 unterscheiden.

Das Grundkonzept sieht jeweils ein Halteprofil 3 mit einer Verriegelungsnut 4 vor sowie ein komplementäres Halteprofil 3', das mit einer Verriegelungsfeder 5 an der gegenüberliegenden Paneelkante 1' versehen ist. Die Verriegelungsnut 4 und die Verriegelungsfeder 5 verriegeln die Paneelkanten 1 und 1' stets formschlüssig, und zwar einerseits in einer Richtung senkrecht zur Paneelebene sowie anderseits in einer Richtung, die senkrecht zu den verriegelten Paneelkanten 1/1' und gleichzeitig parallel zur Paneelebene liegt (horizontal).

Die Verriegelungsnut 4 hat eine obere Nutwand 4a und eine untere Nutwand 4b, welche länger ist als die obere Nutwand. Die untere Nutwand 4b trägt an ihrem freien Ende einen Halterand 11, der sich nach oben in Richtung der Paneeloberfläche S erstreckt.

Für die horizontale Verriegelung sind untere Anschlagflächen vorgesehen, nämlich eine untere Anschlagfläche 10 am Halterand 11 der unteren Nutwand 4b der Verriegelungsnut 4 sowie eine untere Anschlagfläche 12 an der Federunterseite 13 der Verriegelungsfeder 5. Zu diesem Zweck ist an der Federunterseite 13 eine Hinterschneidung 14 vorgesehen, die eine rückwärtige zum Kern des Paneels gerichtete Seite aufweist, an der sich die untere Anschlagfläche 12 der Verriegelungsfeder 5 befindet.

Sowohl die untere Anschlagfläche 12 der Verriegelungsfeder 5 als auch die untere Anschlagfläche 10 der Verriegelungsnut 4 erstrecken sich bei den Beispielen der Figurengruppen 12 bis 15 jeweils senkrecht zur Paneeloberfläche S beziehungsweise S'.

Die Federunterseite 13 hat in den Figurengruppen 12 bis 15 immer eine ebene Kontaktfläche 13a, die sich parallel zur Paneeloberfläche S' erstreckt und die untere Nutwand 4b hat eine Innenseite 19 mit einer Auflagefläche 19a, die sich ebenfalls parallel zur Paneeloberfläche S' erstreckt. An den beiden Enden dieser Kontaktfläche 13a schließen sich jeweils ansteigende Bereiche der Federunterseite 13 an. Ein vorderer ansteigender Bereich 13b steigt zum freien Ende der Verriegelungsfeder 5 an. Ein hinterer Bereich 13d steigt bis zur unteren Anschlagfläche 12 der Verriegelungsfeder 5 an. Der hintere Bereich 13d lässt sich auf einer Kurve an dem Halterand 11 der unteren Nutwand 4b der Verriegelungsnut 4 vorbei bewegen (schwenken), ohne einen Druck gegen den Halterand 11 auszuüben. Auf diese Weise können die Paneelkanten 1 und 1' sehr einfach, und ohne die Halteprofile 3 und 3' elastisch verformen zu müssen, zusammengefügt oder voneinander getrennt werden. Zu diesem Zweck genügt es, das Paneel mit der Verriegelungsfeder 5 aufwärts zu schwenken, und zwar um den Schnittpunkt Z an der Spitze der V-Fuge. Idealerweise liegt deswegen die Kontur des hinteren ansteigenden Bereichs 13d der Federunterseite auf einem Radius R, dessen Mittelpunkt der Schnittpunkt Z der V-Fuge ist.

Falls aber ein gewisses Maß an elastischer Verformung der Halteprofile gewünscht ist, kann an dem hinteren Bereich 13d der Federunterseite 13 einfach etwas mehr Material belassen werden, so dass die Kontur des hinteren ansteigenden Bereichs 13d dann auf einem größeren Radius liegt. Um die Verriegelungsfeder 5 dann in die Verriegelungsnut 4 einzufügen, muss in gewissem Maße eine Kraft auf den Halterand 11 der unteren Nutwand 4b ausgeübt werden, damit er sich zumindest temporär elastisch nach unten biegt und anschließend sich wieder ganz oder teilweise zurückstellt in Richtung seiner neutralen Position.

Der vordere ansteigende Bereich 13b der Federunterseite 13 begünstigt es, die Verriegelungsfeder 5 durch eine Schwenkbewegung M in die Verriegelungsnut 4 einzufügen oder aus dieser heraus zu schwenken, ohne mit der Federunterseite 13 einen wesentlichen Druck auf die untere Nutwand 4b auszuüben.

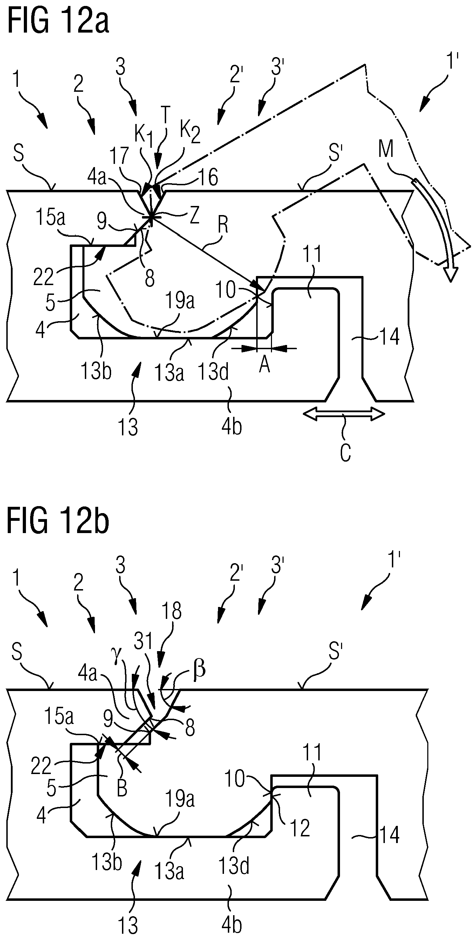

In Fig. 12b sowie in den mit "b" indizierten Darstellungen der folgenden Figurengruppen ist jeweils eine Position der Paneele gezeigt, bei der sich die unteren Anschlagflächen 10 und 12 berühren.

Des Weiteren sind obere Stoßflächen 8 und 9 vorgesehen, die einander dann berühren, wenn die Paneelkanten 1 und 1' aufeinander zu bewegt werden, bis sich zwischen den unteren Anlageflächen 10 und 12 ein maximaler Abstand gebildet hat, wie in Fig. 12a gezeigt. Die oberen Stoßflächen 8 und 9 bilden dann eine geschlossene Stoßfuge T, und der Kontakt der Stoßflächen 8 und 9 definiert ein Bereichsende eines Bewegungsspielraums X für die verriegelten Paneelkanten 1 und 1'. Das andere Bereichsende des Bewegungsspielraums wird durch den Kontakt der unteren Anschlagflächen 10 und 12 definiert, wie in Fig. 12b dargestellt.

Die Kontaktfläche 13a der Federunterseite 13 und die Auflagefläche 19a der unteren Nutwand 4b der Verriegelungsnut 4 dienen als Lager- und Gleitflächen für die relative Bewegung der Paneelkanten 1 und 1' innerhalb des vorgegebenen Bewegungsspielraums X.

Wie erwähnt, unterscheiden sich die Ausführungsbeispiele der Figurengruppen 12 bis 15 durch die Gestaltung der Kantenbrechung und der oberen Stoßflächen.

Gemäß den Figurengruppen 12 bis 15 ist die Kantenbrechung jeweils als Fase ausgebildet. Im verriegelten Zustand bilden die Fasen 16 beziehungswiese 17 eine V-Fuge 18. Unterhalb der V-Fuge befinden sich jeweils die oberen Stoßflächen 8 und 9, die parallel zueinander angeordnet sind und einander berühren können, wie in den Figuren 12a, 13a, 14a und 15a gezeigt.

In Fig. 12a ist die Fase 16 derjenigen Paneelkante 1' welche die Verriegelungsfeder 5 aufweist, gegenüber der Paneeloberfläche S' um einen Winkel β von ca. 60° geneigt, während die obere Stoßfläche 9 dieser Paneelkante um einen kleineren Winkel γ von ca. 45° gegenüber der Paneeloberfläche S' geneigt ist. Wegen der unterschiedlichen Neigung ist zwischen der Fase 16 und der oberen Stoßfläche 9 ein leichter Knick 31 vorhanden. An dem komplementären Halteprofil 3 mit der Verriegelungsnut 4 ist die obere Stoßfläche 8 so geneigt, dass sie sich im verriegelten Zustand zweier Paneelkanten parallel zur gegenüberliegenden oberen Stoßfläche 9 erstreckt. Die obere Stoßfläche 9 erstreckt sich mit dieser Neigung bis an die Innenseite 22 der oberen Nutwand 4a, die parallel zur Paneeloberfläche S ist.

Des Weiteren zeigt Fig. 12a die Paneelkanten 1 und 1' in einer Position, in der sie gegeneinander gestoßen sind, so dass sich die oberen Stoßflächen 8 und 9 berühren. Gleichzeitig haben die unteren Anschlagflächen 10 und 12 in dieser Position einen maximale Abstand A zueinander. In Fig. 12b sind die Paneelkanten 1 und 1' so auseinander bewegt, dass sich zwischen den oberen Stoßflächen 8 und 9 ein maximaler Spalt B gebildet hat. Dadurch sind gleichzeitig die unteren Anlageflächen 10 und 12 miteinander in Kontakt gekommen. Erkennbar ist der zwischen den unteren Anschlagflächen mögliche maximale Abstand A stets größer als die maximale Weite des Spaltes B zwischen den oberen Stoßflächen 8 und 9. Wegen der geneigten Anordnung der oberen Stoßflächen 8 und 9 kann Schmutz, der in die darüber befindliche V-Fuge gelangt, aufgrund der Enge des Spaltes B schlechter in diesen eindringen. Darüber hinaus verhindert die schräge Anordnung der oberen Stoßflächen 8 und 9, dass tief in den Spalt B hinunter geblickt werden kann. Ein Betrachter kann überhaupt nur eine der beiden oberen Stoßflächen 9 zu einem kleinen Teil erblicken, weil die andere Stoßfläche 8 für einen Betrachter stets verdeckt ist. An dem sichtbaren Teil der Stoßfläche 9 bleibt aber die Blicktiefe gering.

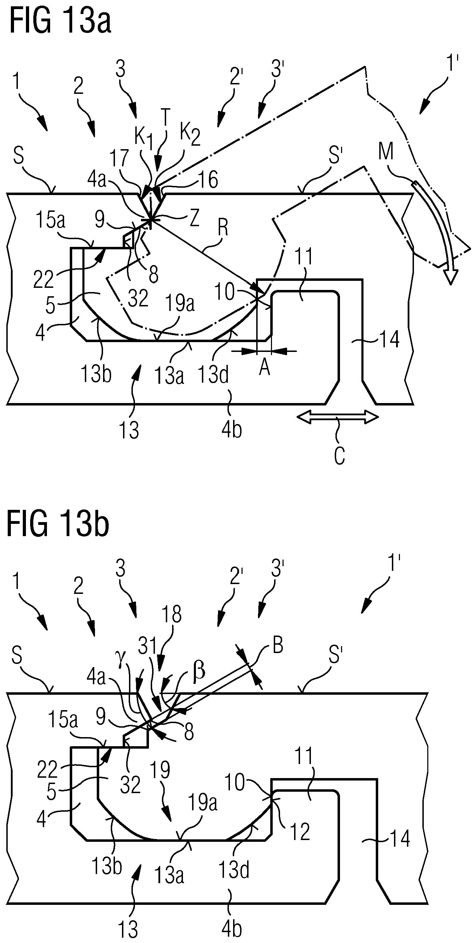

Bei dem Ausführungsbeispiel gemäß Fig. 13a und 13b hat die Fase 16 derjenigen Paneelkante, welche die Verriegelungsfeder 5 aufweist, denselben Neigungswinkel β, wie in Fig. 12a. Die oberen Stoßflächen 8 und 9 sind jedoch um einen Winkel γ gegenüber der Paneeloberfläche S/S' geneigt, der noch etwas kleiner ist als der Winkel γ in Fig. 12a. Dadurch ist die Weite des Spaltes B enger als im vorherigen Ausführungsbeispiel. Zwischen der oberen Stoßfläche 8 und 9 und demjenigen Bereich der Innenseite 22 der oberen Nutwand 4a, der parallel zur Paneeloberfläche S ist, erstreckt sich ein kurzer Abschnitt senkrecht zur Paneeloberfläche S, so dass sich ein kleiner Absatz 32 bildet. Wenn der Absatz 32 nicht vorhanden wäre und die obere Stoßfläche 8 mit gleichbleibender Neigung bis an die Innenseite 22 der oberen Nutwand 4a heranreichte, dann würde der Flächenkontakt zwischen der horizontalen Federoberseite 15a und dem horizontalen Bereich der oberen Nutwand 4a deutlich verringert. Deshalb dient der Absatz 32 dazu, den horizontalen Flächenkontakt an dieser Stelle zu erhöhen und auf diese Weise die Festigkeit der Verriegelung zu verbessern.

Der in Fig. 13a dargestellte Abstand A zwischen den unteren Anschlagflächen 10 und 12 ist identisch mit Fig. 12a. Der in Fig. 13b dargestellte Spalt B ist jedoch enger als in Fig. 12b, weil die oberen Stoßflächen 8 und 9 mit einem kleineren Neigungswinkel zur Paneeloberfläche S angeordnet sind als in Fig. 12b.

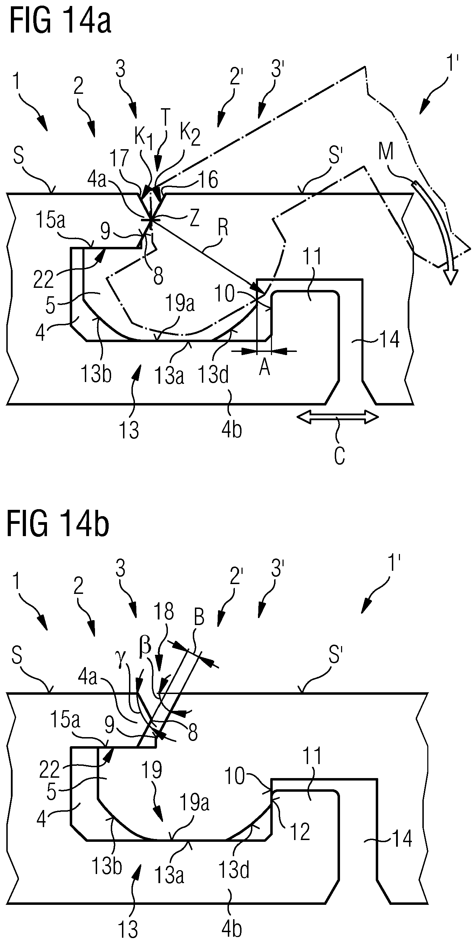

Die Figuren 14a und 14b zeigen ein Ausführungsbeispiel bei dem die Fase 16 derjenigen Paneelkante 1', welche die Verriegelungsfeder 5 aufweist, wiederum denselben Neigungswinkel β hat wie in Fig. 12a. Die oberen Stoßflächen 8 und 9 sind jedoch um einen Winkel γ gegenüber der Paneeloberfläche S/S' geneigt, der identisch ist mit dem Neigungswinkel β der Fase 16. Das heißt, bei derjenigen Paneelkante, welche die Verriegelungsfeder 5 hat, sind die Fase 16 und die obere Stoßfläche 9 in einer gemeinsamen Ebene angeordnet. Daher ist bei dieser Paneelkante 1' zwischen Fase 16 und Stoßfläche 9 kein Knick vorhanden.

Die komplementäre obere Stoßfläche 8 an der oberen Nutwand 4a derjenigen Paneelkante mit der Verriegelungsnut 4 erstreckt sich mit gleichbleibender Neigung bis an die horizontale Innenseite 22 der oberen Nutwand 4a heran.

Der in Fig. 14a dargestellte Abstand A zwischen den unteren Anschlagflächen 10 und 12 ist identisch mit Fig. 12a. Der in Fig. 14b dargestellte Spalt B hat jedoch eine größere Weite als in Fig. 12b, weil die oberen Stoßflächen 8 und 9 mit einem größeren Neigungswinkel zur Paneeloberfläche S angeordnet sind als in Fig. 12b.

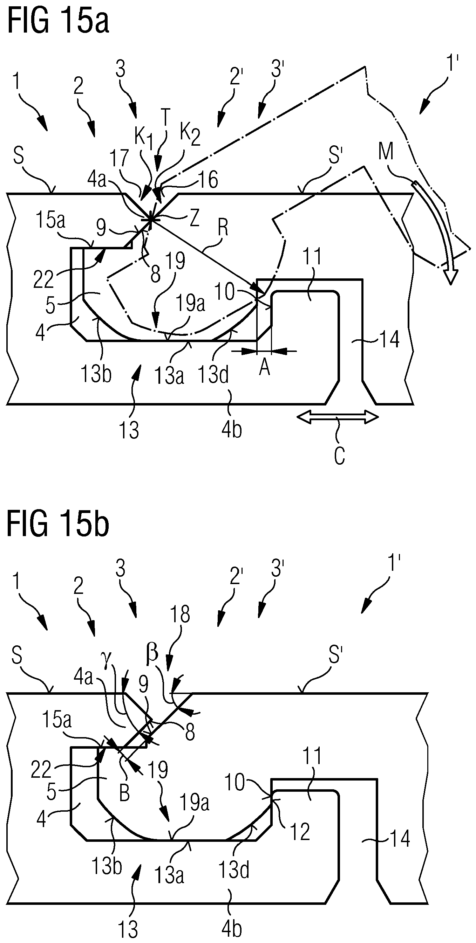

Das Ausführungsbeispiel der Figuren 15a und 15b unterscheidet sich von dem vorherigen Ausführungsbeispiel 14a/14b lediglich durch den geänderten Winkel der Fasen 16 und 17. Diese sind gegenüber der Paneeloberfläche S/S' in einem geringeren Winkel β geneigt, der hier ungefähr 45° beträgt. Bei derjenigen Paneelkante, welche die Verriegelungsfeder 5 hat, sind die Fase 16 und die obere Stoßfläche 9 wiederum in einer gemeinsamen Ebene angeordnet. Das heißt, die obere Stoßfläche 9 dieser Paneelkante 1' ist um einen Winkel γ gegenüber der Paneeloberfläche S' geneigt, der mit dem Neigungswinkel β der Fase 16 identisch ist.

Der in Fig. 15a dargestellte Abstand A zwischen den unteren Anschlagflächen 10 und 12 ist wiederum identisch mit Fig. 12a. Der in Fig. 15b dargestellte Spalt B hat die gleiche Weite wie Spalt B in Fig. 12b, weil der Neigungswinkel γ der oberen Stoßflächen 8 und 9 identisch ist.

Die Weite des Spalt B liegt damit zwischen derjenigen in Fig. 13b, welcher enger ist und der Spaltweite B gemäß Fig. 14b, die größer ist.

### Bezugszeichenliste

- 1: Paneelkante
- 1': Paneelkante
- 2: Verriegelungsmittel
- 2': Verriegelungsmittel
- 3: Halteprofil
- 3': Halteprofil
- 4: Verriegelungsnut
- 4a: obere Nutwand
- 4b: untere Nutwand
- 5: Verriegelungsfeder
- 6: Paneelplatte
- 7: separater Streifen
- 8: obere Stoßfläche (Verriegelungsnut)
- 9: obere Stoßfläche (Verriegelungsfeder)
- 10: untere Anschlagfläche (Verriegelungsnut)
- 11: Halterand
- 12: untere Anschlagfläche (Verriegelungsfeder)
- 13: Federunterseite
- 13a: Kontaktfläche
- 13b: ansteigender vorderer Bereich
- 13c: Absatz
- 13d: ansteigender hinterer Bereich
- 14: Hinterschneidung
- 15: Federoberseite
- 15a: horizontaler Abschnitt
- 16: Fase
- 17: Fase
- 18: V-Fuge
- 19: Innenseite (untere Nutwand)
- 19a: Auflagefläche (untere Nutwand)
- 19b: aufsteigender Bereich
- 19c: Aussparung
- 20: Verschlussfeder
- 20a: Federspitze
- 20b: untere Seitenfläche
- 21: Verschlussnut
- 22: Innenseite (obere Nutwand)
- 23: Knick
- 24: Vorderbereich (Federunterseite)
- 24a: gekrümmte Kontaktfläche
- 25: Auflagefläche (Verriegelungsnut)
- 25a: Aufstiegsbereich
- 26: hinterer Bereich (Federunterseite)
- 27: gewölbter Vorsprung
- 28: Vertiefung (Auflagefläche)
- 29: Hohlraum
- 30: Freiraum
- 31: Knick
- 32: Absatz
- C: Doppelpfeil
- K₁: Kantenbrechung
- K₂: Kantenbrechung
- M: Schwenkbewegung
- R: Radius
- S: Paneeloberfläche
- S': Paneeloberfläche
- T: Stoßfuge
- Z: Schnittpunkt
- α: Winkel
- β: Winkel
- γ: Winkel

## Patentansprüche

1. Paneel mit wenigstens einem Paar komplementärer Verriegelungsmittel (2, 2') an gegenüberliegenden Paneelkanten (1, 1'), wobei die Verriegelungsmittel (2, 2') als formschlüssige Halteprofile (3, 3') mit Verriegelungsnut (4) beziehungsweise mit komplementärer Verriegelungsfeder (5) ausgestaltet sind, wobei die Paneeloberfläche (S, S') wenigstens am Rand eines der Halteprofile (3, 3') eine Kantenbrechung (K₁, K₂) aufweist, mit der Maßgabe, dass jenes mit der Kantenbrechung (K₁, K₂) versehene Halteprofil (3, 3') unterhalb der Kantenbrechung (K₁, K₂) mit einer oberen Stoßfläche (8, 9) versehen ist, und das komplementäre Halteprofil (3, 3') mit einer dazu im Wesentlichen parallel angeordneten komplementären oberen Stoßfläche (8, 9) versehen ist, und wobei durch die beiden Stoßflächen (8, 9) im Kontakt miteinander eine Stoßfuge (T) erzeugbar ist, wobei die Stoßfuge (T) relativ zur Paneeloberfläche (S, S') und gegenüber dem Lot auf der Paneeloberfläche geneigt ist und zu diesem Zweck eine der Stoßflächen (8, 9) einer Feder (5, 20) zugeordnet ist und in Richtung des freien Endes der betreffenden Feder (5, 20) abwärts geneigt ist und die komplementäre obere Stoßfläche (8, 9) einer Nut (4, 21) zugeordnet ist und in Richtung des Grundes der betreffenden Nut (4, 21) abwärts geneigt ist, wobei die komplementären Halteprofile (3, 3') so gestaltet sind, dass sie sich durch eine Schwenkbewegung (M) formschlüssig verbinden lassen, wobei, dass an der Paneeloberfläche (S, S') zweier verbundener Paneelkanten (1, 1') eine geschlossene Stoßfuge (T) erzeugbar ist, mit der Maßgabe, dass durch die beiden Stoßflächen (8, 9) im Kontakt miteinander ein erstes Bereichsende eines Bewegungsspielraums (X) definiert ist, und zwar für eine Bewegung der Paneelkanten relativ zueinander und in einer Bewegungsrichtung, die sowohl senkrecht zu den formschlüssig verbundenen Paneelkanten als auch parallel zur Ebene der verbundenen Paneele liegt, wobei jedes der Halteprofile (3, 3') je eine untere Anschlagfläche (10, 12) aufweist, welche dann maximal voneinander beabstandet sind, wenn die oberen Stoßflächen (8, 9) einander berühren und die Stoßfuge (T) geschlossen ist, wobei der maximale Abstand zwischen den unteren Anschlagflächen (10, 12) die Größe des Bewegungsspielraums (X) bemisst, wobei die unteren Anschlagflächen (10, 12), wenn sie einander berühren, das zweite Bereichsende des Bewegungsspielraums (X) definieren, und dass eine Federunterseite (13) der Verriegelungsfeder (5) eine untere Kontaktfläche (13a) aufweist und eine untere Nutwand (4b) der Verriegelungsnut (4) mit einer Auflagefläche (19a) für die untere Kontaktfläche (13a) der Verriegelungsfeder (5) versehen ist, **dadurch gekennzeichnet, dass** die untere Kontaktfläche (13a) der Verriegelungsfeder (5) parallel zur Paneeloberfläche (S, S') angeordnet ist und sich die Auflagefläche (19a) ebenfalls parallel zur Paneeloberfläche (S, S') erstreckt und dass die Auflagefläche (19a) für die untere Kontaktfläche (13a) in Gebrauchslage horizontal angeordnet ist.

2. Paneel nach Anspruch 1, **dadurch gekennzeichnet, dass** an dem Rand des Halteprofils (3'), das die Verriegelungsfeder (5) aufweist, eine Kantenbrechung (K₂) vorgesehen ist, dass die Kantenbrechung (K₂) als Fase (16) ausgebildet ist, dass die Fase (16) zum freien Ende der Verriegelungsfeder hin abwärts geneigt ist, und dass die obere Stoßfläche (9) dieses Halteprofils (3') ebenfalls zum freien Ende der Verriegelungsfeder (5) hin abwärts geneigt ist, wobei der Neigungswinkel der oberen Stoßfläche (9) kleiner oder gleich groß oder größer als der Neigungswinkel der Fase (16) ist.

3. Paneel nach Anspruch 1 oder 2, **dadurch gekennzeichnet, dass** eine Federoberseite (15) der Verriegelungsfeder (5) in Bezug zur Paneeloberfläche (S, S') geneigt ist und die Federoberseite (15) und die obere Stoßfläche (9) zu einer gemeinsamen Fläche integriert sind, dass die Verriegelungsnut (4) an der Innenseite (22) ihrer oberen Nutwand (4a) ebenfalls in Bezug zur Paneeloberfläche (S, S') geneigt ist, und dass deren Neigung angepasst ist an die Neigung der oberen Stoßfläche (9) der Verriegelungsfeder.

4. Paneel nach einem der Ansprüche 1 bis 3, **dadurch gekennzeichnet, dass** die Verriegelungsnut (4) eine untere Nutwand (4b) hat, an deren freiem Ende ein zur Paneeloberseite (S, S') gerichteter Halterand (11) vorgesehen ist, dass die untere Anschlagfläche (10) der Verriegelungsnut (4) an dem Halterand (11) der unteren Nutwand (4b) angeordnet ist, und dass das Lot auf der unteren Anschlagfläche (10) nach innen zum Nutgrund der Verriegelungsnut (4) gerichtet ist.

5. Paneel nach einem der Ansprüche 1 bis 4, **dadurch gekennzeichnet, dass** von den unteren Anschlagflächen (10, 12) zumindest die untere Anschlagfläche (10) der Verriegelungsnut (4) in Bezug zur Paneeloberfläche (S, S') senkrecht angeordnet ist.

6. Paneel nach Anspruch 5, **dadurch gekennzeichnet, dass** die Federunterseite (13) der Verriegelungsfeder (5) zumindest an einem der beiden Enden der unteren Kontaktfläche (13a) einen ansteigenden Bereich (13a, 13d) aufweist.

7. Paneel nach einem der Ansprüche 1 bis 6, **dadurch gekennzeichnet, dass** das Halteprofil (3') mit der Verriegelungsfeder (5) oberhalb der Verriegelungsfeder (5) eine Verschlussnut (21) aufweist, und dass das komplementäre Halteprofil (3) mit Verriegelungsnut (4) mit seinem freien Ende der oberen Nutwand (4a) eine Verschlussfeder (20) bildet, welche in die Verschlussnut (21) einfügbar ist.

8. Paneel nach Anspruch 7, **dadurch gekennzeichnet, dass** die obere Stoßfläche (8) derjenigen Paneelkante (1), welche mit der Verschlussfeder (20) versehen ist, an der Federoberseite der Verschlussfeder (20) angeordnet ist, und dass die obere Stoßfläche (9) derjenigen Paneelkante (1'), welche mit der Verschlussnut (21) versehen ist, an der oberen Nutwand der Verschlussnut (21) angeordnet ist.

## Claims

1. A panel comprising at least one pair of complementary locking means (2, 2') at mutually opposite panel edges (1, 1'), wherein the locking means (2, 2') are in the form of positively locking holding profiles (3, 3') with a locking groove (4) and with a complementary locking tongue (5) respectively, wherein at least at the edge of one of the holding profiles (3, 3') the panel surface (S, S') has an edge break portion (K₁, K₂), with the proviso that that holding profile (3, 3') which is provided with the edge break portion (K₁, K₂) is provided with an upper contact surface (8, 9) beneath the edge break portion (K₁, K₂) and the complementary holding profile (3, 3') is provided with a complementary upper contact surface (8, 9) which is arranged substantially parallel thereto, and wherein a butt joint (T) can be produced by the two contact surfaces (8, 9) in contact with each other, wherein the butt joint (T) is inclined relative to the panel surface (S, S') and relative to the perpendicular to the panel surface and for that purpose one of the contact surfaces (8, 9) is associated with a tongue (5, 20) and is inclined downwardly in the direction of the free end of the tongue (5, 20) in question and the complementary upper contact surface (8, 9) is associated with a groove (4, 21) and is inclined downwardly in the direction of the bottom of the groove (4, 21) in question, the complementary holding profiles (3, 3') are so designed that they can be positively lockingly connected by a pivotal movement (M), wherein a closed butt joint (T) can be produced at the panel surface (S, S') of two connected panel edges (1, 1'), with the proviso that a first range end of a scope for movement (X) is defined by the two contact surfaces (8, 9) in contact with each other, and more specifically for a movement of the panel edges relative to each other and in a direction of movement which is both perpendicular to the positively lockingly connected panel edges and also parallel to the plane of the connected panels, wherein each of the holding profiles (3, 3') has a respective lower abutment surface (10, 12) which are then spaced from each other at the maximum when the upper contact surfaces (8, 9) are in contact with each other and the butt joint (T) is closed, wherein the maximum spacing between the lower abutment surfaces (10, 12) measures the size of the scope for movement (X), wherein the lower abutment surfaces (10, 12) when they are in contact with each other define the second range end of the scope for movement (X), and that an underside (13) of the locking tongue (5) has a lower contacting surface (13a) and a lower groove wall (4b) of the locking groove (4) is provided with a support surface (19a) for the lower contacting surface (13a) of the locking tongue (5), **characterised in that** the lower contacting surface (13a) of the locking tongue (5) is arranged parallel to the panel surface (S, S') and the support surface (19a) also is arranged parallel to the panel surface (S, S') and that the support surface (19a) for the lower contacting surface (13a) in the position of use is arranged horizontally.

2. A panel as set forth in claim 1 **characterised in that** at the edge of the holding profile (3') which has the locking tongue (5) there is provided an edge break portion (K₂), that the edge break portion (K₂) is in the form of a chamfer (16), that the chamfer (16) is inclined downwardly towards the free end of the locking tongue and that the upper contact surface (9) of said holding profile (3') is also inclined downwardly towards the free end of the locking tongue (5), wherein the angle of inclination of the upper contact surface (9) is less than or equal to or larger than the angle of inclination of the chamfer (16).

3. A panel as set forth in claim 1 or 2 **characterised in that** a tongue top side (15) of the locking tongue (5) is inclined in relation to the panel surface (S, S') and the tongue top side (15) and the upper contact surface (9) are integrated to constitute a common surface, that the locking groove (4) at the inside (22) of its upper groove wall (4a) is also inclined in relation to the panel surface (S, S') and that the inclination thereof is matched to the inclination of the upper contact surface (9) of the locking tongue.

4. A panel as set forth in one of claims 1 through 3 **characterised in that** the locking groove (4) has a lower groove wall (4b), at the free end of which there is provided a holding edge (11) directed towards the panel top side (S, S'), that the lower abutment surface (10) of the locking groove (4) is arranged at the holding edge (11) of the lower groove wall (4b) and that the perpendicular on the lower abutment surface (10) is directed inwardly towards the bottom of the locking groove (4).

5. A panel as set forth in one of claims 1 through 4 **characterised in that** of the lower abutment surfaces (10, 12) at least the lower abutment surface (10) of the locking groove (4) is arranged perpendicularly in relation to the panel surface (S, S').

6. A panel as set forth in claim 5 **characterised in that** the tongue underside (13) of the locking tongue (5), at least at one of the two ends of the lower contacting surface (13a), has a rising region (13a, 13d).

7. A panel as set forth in one of claims 1 through 6 **characterised in that** the holding profile (3') with the locking tongue (5) has a closure groove (21) above the locking tongue (5) and the complementary holding profile (3) with the locking groove (4) with its free end of the upper groove wall (4a) forms a closure tongue (20) which can be inserted into the closure groove (21).

8. A panel as set forth in claim 7 **characterised in that** the upper contact surface (8) of that panel edge (1) which is provided with the closure tongue (20) is arranged at the tongue top side of the closure tongue (20) and the upper contact surface (9) of that panel edge (1') which is provided with the closure groove (21) is arranged at the upper groove wall of the closure groove (21).

## Revendications

1. Panneau ayant au moins une paire de moyens de verrouillage complémentaires (2, 2') sur des chants opposés du panneau (1, 1'), les moyens de verrouillage (2, 2') étant configurés comme profilés de maintien à complémentarité de forme (3, 3') avec rainure de verrouillage (4) respectivement avec languette de verrouillage complémentaire (5), la surface du panneau (S, S') présentant au moins au bord d'un des profilés de maintien (3, 3') un biseautage de chant (K₁, K₂), sous réserve que le profilé de maintien (3, 3') à biseautage de chant (K₁, K₂) soit muni en dessous du biseautage de chant (K₁, K₂) d'une surface d'about supérieure (8, 9) et que le profilé de maintien complémentaire (3, 3') soit muni d'une surface d'about supérieure complémentaire (8, 9) disposée essentiellement parallèlement à elle, les deux surfaces d'about (8, 9), quand elles sont en contact entre elles, pouvant créer un joint d'about (T), le joint d'about (T) étant incliné relativement à la surface du panneau (S, S') et par rapport à la perpendiculaire de la surface du panneau et, à cet effet, une des surfaces d'about (8, 9) étant affectée à une languette (5, 20) et inclinée vers le bas dans le sens de l'extrémité libre de la languette concernée (5, 20) et la surface d'about supérieure complémentaire (8, 9) étant affectée à une languette (4, 21) et inclinée vers le bas dans le sens du fond de la rainure concernée (4, 21), les profilés de maintien complémentaires (3, 3') étant configurés de sorte à pouvoir être assemblés par complémentarité de forme selon un mouvement pivotant (M), un joint d'about fermé (T) étant réalisable sur la surface du panneau (S, S') de deux chants de panneau assemblés (1, 1'), sous réserve que les deux surfaces d'about (8, 9), quand elles sont en contact entre elles, définissent une première extrémité de plage d'un jeu (X), et ce pour un déplacement des chants de panneau l'un par rapport à l'autre et dans un sens de déplacement qui est à la fois perpendiculaire aux chants de panneau assemblés par complémentarité de forme et parallèle au plan des panneaux assemblés, chacun des profilés de maintien (3, 3') présentant une surface de butée inférieure (10, 12), qui sont espacées entre elles au maximum quand les surfaces d'about supérieures (8, 9) se touchent et que le joint d'about (T) est fermé, l'écart maximum entre les surfaces de butée inférieures (10, 12) définissant la taille du jeu (X), les deux surfaces de butée inférieures (10, 12), quand elles se touchent, définissant la deuxième extrémité de plage du jeu (X), et qu'une face inférieure de languette (13) de la languette de verrouillage (5) présente une surface de contact inférieure (13a) et qu'une paroi inférieure de rainure (4b) de la rainure de verrouillage (4) soit munie d'une surface d'appui (19a) pour la surface de contact inférieure (13a) de la languette de verrouillage (5), **caractérisé en ce que** la surface de contact inférieure (13a) de la languette de verrouillage (5) est disposée parallèlement à la surface du panneau (S, S') et que la surface d'appui (19a) s'étend également parallèlement à la surface du panneau (S, S') et que la surface d'appui (19a) pour la surface de contact inférieure (13a) est disposée horizontalement en position d'utilisation.

2. Panneau selon la revendication 1, **caractérisé en ce qu'**un biseautage de chant (K₂) est prévu au niveau du bord du profilé de maintien (3') muni de la languette de verrouillage (5), que le biseautage de chant (K₂) est configuré en tant que chanfrein (16), que le chanfrein (16) est incliné vers le bas vers l'extrémité libre de la languette de verrouillage et que la surface d'about supérieure (9) de ce profilé de maintien (3') est également inclinée vers le bas vers l'extrémité libre de la languette de verrouillage (5), l'angle d'inclinaison de la surface d'about supérieure (9) étant inférieur ou égal à l'angle d'inclinaison du chanfrein (16).

3. Panneau selon la revendication 1 ou 2, **caractérisé en ce qu'**une face supérieure de languette (15) de la languette de verrouillage (5) est inclinée par rapport à la surface du panneau (S, S') et que la face supérieure de la languette (15) et la surface d'about supérieure (9) sont intégrées pour former une surface commune, que la rainure de verrouillage (4) est également inclinée à l'intérieur (22) de sa paroi de rainure supérieure (4a) par rapport à la surface du panneau (S, S') et que son inclinaison est adaptée à l'inclinaison de la surface d'about supérieure (9) de la languette de verrouillage.

4. Panneau selon une des revendications 1 à 3, **caractérisé en ce que** la languette de verrouillage (4) a une paroi inférieure de rainure (4b) à l'extrémité libre de laquelle est prévu un rebord de maintien (11) orienté vers la surface du panneau (S, S'), que la surface de butée inférieure (10) de la rainure de verrouillage (4) est disposée au niveau du rebord de maintien (11) de la paroi inférieure de rainure (4b) et que la perpendiculaire à la surface de butée inférieure (10) est orientée vers l'intérieur vers le fond de la rainure de verrouillage (4).

5. Panneau selon une des revendications 1 à 4, **caractérisé en ce que** parmi les surfaces de butée inférieures (10, 12), au moins la surface de butée inférieure (10) de la rainure de verrouillage (4) est disposée perpendiculairement à la surface du panneau (S, S').

6. Panneau selon la revendication 5, **caractérisé en ce que** la face inférieure de la languette (13) de la languette de verrouillage (5) présente, au moins à l'une des deux extrémités de la surface de contact inférieure (13a), une zone montante (13a, 13d).

7. Panneau selon une des revendications 1 à 6, **caractérisé en ce que** le profilé de maintien (3') avec la languette de verrouillage (5) présente au dessus de la languette de verrouillage (5) une rainure de fermeture (21) et que le profilé de maintien complémentaire (3) avec rainure de verrouillage (4) constitue avec son extrémité libre de la paroi supérieure de la rainure (4a) une languette de fermeture (20) qui est insérable dans la rainure de fermeture (21).

8. Panneau selon la revendication 7, **caractérisé en ce que** la surface d'about supérieure (8) du chant du panneau (1) muni de la languette de fermeture (20) est disposée sur la face supérieure de la languette de fermeture (20) et que la surface d'about supérieure (9) du chant du panneau (1') muni de la rainure de fermeture (21) est disposée au niveau de la paroi supérieur de la rainure de fermeture (21).
